(19) Europäisches Patentamt
European Patent Office
Office européen des brevets

(11) EP 0 551 329 B1

(12) EUROPEAN PATENT SPECIFICATION

(45) Date of publication and mention
of the grant of the patent:
**15.07.1998 Bulletin 1998/29**

(51) Int Cl.$^6$: **C12N 15/15**, C07K 14/81,
A61K 38/55

(21) Application number: **91917143.9**

(22) Date of filing: **01.10.1991**

(86) International application number:
**PCT/DK91/00299**

(87) International publication number:
**WO 92/06111 (16.04.1992 Gazette 1992/09)**

(54) **APROTININ ANALOGUES**

APROTININANALOGA

ANALOGUES D'APROTININE

(84) Designated Contracting States:
**AT BE CH DE DK ES FR GB GR IT LI LU NL SE**

(30) Priority: **01.10.1990 DK 236190**
**12.06.1991 DK 111891**

(43) Date of publication of application:
**21.07.1993 Bulletin 1993/29**

(73) Proprietor: **NOVO NORDISK A/S**
**2880 Bagsvaerd (DK)**

(72) Inventors:
 • **BJORN, Soren, Erik**
 **DK-2800 Lyngby (DK)**
 • **NORRIS, Kjeld**
 **DK-2900 Hellerup (DK)**

 • **DINESS, Viggo**
 **DK-2920 Charlottenlund (DK)**
 • **NORSKOV-LAURITSEN, Leif Birkevej 10**
 **DK-4600 Koge (DK)**
 • **CHRISTENSEN, Niels, Dyhr**
 **DK-2300 Kobenhavn S (DK)**
 • **BREGENGAARD, Claus**
 **DK-2900 Hellerup (DK)**

(74) Representative: **Hegner, Mogens et al**
**c/o Hofman-Bang & Boutard, Lehmann & Ree A/S,**
**Hans Bekkevolds Allé 7**
**2900 Hellerup (DK)**

(56) References cited:
**EP-A- 0 238 993**         **EP-A- 0 297 362**
**EP-A- 0 339 942**         **EP-A- 0 419 878**

SEQUENCE LISTING

(1) GENERAL INFORMATION:

(i) APPLICANT:
Bjoern, Soeren Erik
Norris, Kjeld
Diness, Viggo
Noerskov-Lauritsen, Leif

Christensen, Niels Dyhr
Bregengaard, Claus

(ii) TITLE OF INVENTION: Aprotinin Analogues

(iii) NUMBER OF SEQUENCES: 29

(iv) CORRESPONDENCE ADDRESS:

    (A) ADDRESSEE: Novo Nordisk A/S
    (B) STREET: Novo Allé
    (C) CITY: Bagsvaerd
    (E) COUNTRY: Denmark
    (F) ZIP: 2880

(v) COMPUTER READABLE FORM:

    (A) MEDIUM TYPE: Floppy disk
    (B) COMPUTER: IBM PC compatible
    (C) OPERATING SYSTEM: PC-DOS/MS-DOS
    (D) SOFTWARE: PatentIn Release #1.0, Version #1.25

(vi) CURRENT APPLICATION DATA:

    (A) APPLICATION NUMBER:
    (B) FILING DATE:
    (C) CLASSIFICATION:

(vii) PRIOR APPLICATION DATA:

    (A) APPLICATION NUMBER: DK 2361/90
    (B) FILING DATE: 01-OCT-1990

(viii) ATTORNEY/AGENT INFORMATION:

    (A) NAME: Thalsoe-Madsen, Birgit
    (C) REFERENCE/DOCKET NUMBER: 3465.204-WO

(ix) TELECOMMUNICATION INFORMATION:

    (A) TELEPHONE: (212) 867-0123
    (B) TELEFAX: (212) 867-0298
    (C) TELEX:

(2) INFORMATION FOR SEQ ID NO:1:

(i) SEQUENCE CHARACTERISTICS:

    (A) LENGTH: 418 base pairs
    (B) TYPE: nucleic acid
    (C) STRANDEDNESS: single
    (D) TOPOLOGY: linear

(ii) MOLECULE TYPE: cDNA

(vi) ORIGINAL SOURCE:

    (A) ORGANISM: synthetic

(ix) FEATURE:

    (A) NAME/KEY: CDS
    (B) LOCATION: 77..409

(ix) FEATURE:

    (A) NAME/KEY: sig_peptide
    (B) LOCATION: 77..235

(ix) FEATURE:

    (A) NAME/KEY: mat_peptide
    (B) LOCATION: 236..409

(xi) SEQUENCE DESCRIPTION: SEQ ID NO:1:

```
GAATTCCATT CAAGAATAGT TCAAACAAGA AGATTACAAA CTATCAATTT CATACACAAT          60

ATAAACGACC AAAAGA ATG AAG GCT GTT TTC TTG GTT TTG TCC TTG ATC           109
               Met Lys Ala Val Phe Leu Val Leu Ser Leu Ile
               -53          -50                  -45

GGA TTC TGC TGG GCC CAA CCA GTC ACT GGC GAT GAA TCA TCT GTT GAG          157
Gly Phe Cys Trp Ala Gln Pro Val Thr Gly Asp Glu Ser Ser Val Glu
        -40              -35                  -30

ATT CCG GAA GAG TCT CTG ATC ATC GCT GAA AAC ACC ACT TTG GCT AAC          205
Ile Pro Glu Glu Ser Leu Ile Ile Ala Glu Asn Thr Thr Leu Ala Asn
        -25              -20                  -15

GTC GCC ATG GCT GAG AGA TTG GAG AAG AGA GAG CCT GAT TTC TGT TTG          253
Val Ala Met Ala Glu Arg Leu Glu Lys Arg Glu Pro Asp Phe Cys Leu
-10              -5                   1                 5

GAA CCT CCA TAC ACT GGT CCA TGT AAA GCT AGA ATC ATC AGA TAC TTC          301
Glu Pro Pro Tyr Thr Gly Pro Cys Lys Ala Arg Ile Ile Arg Tyr Phe
             10              15                   20

TAC AAC GCC GAA GCT GGT TTG TGT CAA ACT TTC GTT TAC GGT GGC TGC          349
Tyr Asn Ala Glu Ala Gly Leu Cys Gln Thr Phe Val Tyr Gly Gly Cys
         25              30                   35

AGA GCT GAA AGA AAC AAC TTC GAA TCT GCT GAA GAC TGC ATG AGA ACT          397
Arg Ala Glu Arg Asn Asn Phe Glu Ser Ala Glu Asp Cys Met Arg Thr
         40              45                   50


TGT GGT GGT GCC TAATCTAGA                                                 418
Cys Gly Gly Ala
      55
```

(2) INFORMATION FOR SEQ ID NO:2:

    (i) SEQUENCE CHARACTERISTICS:

        (A) LENGTH: 111 amino acids
        (B) TYPE: amino acid
        (D) TOPOLOGY: linear

    (ii) MOLECULE TYPE: protein

    (xi) SEQUENCE DESCRIPTION: SEQ ID NO:2:

```
Met Lys Ala Val Phe Leu Val Leu Ser Leu Ile Gly Phe Cys Trp Ala
-53         -50             -45                 -40

Gln Pro Val Thr Gly Asp Glu Ser Ser Val Glu Ile Pro Glu Glu Ser
        -35             -30             -25

Leu Ile Ile Ala Glu Asn Thr Thr Leu Ala Asn Val Ala Met Ala Glu
    -20             -15             -10

Arg Leu Glu Lys Arg Glu Pro Asp Phe Cys Leu Glu Pro Pro Tyr Thr
    -5             1             5                 10

Gly Pro Cys Lys Ala Arg Ile Ile Arg Tyr Phe Tyr Asn Ala Glu Ala
            15              20              25

Gly Leu Cys Gln Thr Phe Val Tyr Gly Gly Cys Arg Ala Glu Arg Asn
        30              35              40

Asn Phe Glu Ser Ala Glu Asp Cys Met Arg Thr Cys Gly Gly Ala
        45              50              55
```

(2) INFORMATION FOR SEQ ID NO:3:

    (i) SEQUENCE CHARACTERISTICS:

        (A) LENGTH: 418 base pairs
        (B) TYPE: nucleic acid
        (C) STRANDEDNESS: single
        (D) TOPOLOGY: linear

    (ii) MOLECULE TYPE: cDNA

    (vi) ORIGINAL SOURCE:

        (A) ORGANISM: synthetic

    (ix) FEATURE:

        (A) NAME/KEY: CDS
        (B) LOCATION: 77..409

    (ix) FEATURE:

        (A) NAME/KEY: sig_peptide

(B) LOCATION: 77..235

(ix) FEATURE:

(A) NAME/KEY: mat_peptide
(B) LOCATION: 236..409

(xi) SEQUENCE DESCRIPTION: SEQ ID NO:3:

```
GAATTCCATT CAAGAATAGT TCAAACAAGA AGATTACAAA CTATCAATTT CATACACAAT          60

ATAAACGACC AAAAGA ATG AAG GCT GTT TTC TTG GTT TTG TCC TTG ATC             109
               Met Lys Ala Val Phe Leu Val Leu Ser Leu Ile
               -53          -50                 -45

GGA TTC TGC TGG GCC CAA CCA GTC ACT GGC GAT GAA TCA TCT GTT GAG          157
Gly Phe Cys Trp Ala Gln Pro Val Thr Gly Asp Glu Ser Ser Val Glu
        -40                 -35                 -30

ATT CCG GAA GAG TCT CTG ATC ATC GCT GAA AAC ACC ACT TTG GCT AAC          205
Ile Pro Glu Glu Ser Leu Ile Ile Ala Glu Asn Thr Thr Leu Ala Asn
        -25                 -20                 -15

GTC GCC ATG GCT GAG AGA TTG GAG AAG AGA GAG CCT GAT TTC TGT TTG          253
Val Ala Met Ala Glu Arg Leu Glu Lys Arg Glu Pro Asp Phe Cys Leu
-10                 -5                  1                   5

GAA CCT CCA TAC ACT GGT CCA TGT AAA GCT AGA ATC ATC AGA TAC TTC          301
Glu Pro Pro Tyr Thr Gly Pro Cys Lys Ala Arg Ile Ile Arg Tyr Phe
                10              15                  20

TAC AAC GCC AAG GCT GGT TTG TGT CAA ACT TTC GTT TAC GGT GGC TGC          349
Tyr Asn Ala Lys Ala Gly Leu Cys Gln Thr Phe Val Tyr Gly Gly Cys
            25                  30                  35

AGA GCT AAG GAA AAC AAC TTC GAA TCT GCT GAA GAC TGC ATG AGA ACT          397
Arg Ala Lys Glu Asn Asn Phe Glu Ser Ala Glu Asp Cys Met Arg Thr
            40                  45                  50

TGT GGT GGT GCC TAATCTAGA                                                 418
Cys Gly Gly Ala
55
```

(2) INFORMATION FOR SEQ ID NO:4:

(i) SEQUENCE CHARACTERISTING:

(A) LENGTH: 111 amino acids
(B) TYPE: amino acid
(C) TOPOLOGY: linear

(ii) MOLECULE TYPE: protein

(xi) SEQUENCE DESCRIPTION: SEQ ID NO:4:

```
Met Lys Ala Val Phe Leu Val Leu Ser Leu Ile Gly Phe Cys Trp Ala
-53          -50              -45              -40

Gln Pro Val Thr Gly Asp Glu Ser Ser Val Glu Ile Pro Glu Glu Ser
        -35              -30              -25

Leu Ile Ile Ala Glu Asn Thr Thr Leu Ala Asn Val Ala Met Ala Glu
        -20              -15              -10

Arg Leu Glu Lys Arg Glu Pro Asp Phe Cys Leu Glu Pro Pro Tyr Thr
-5              1              5              10

Gly Pro Cys Lys Ala Arg Ile Ile Arg Tyr Phe Tyr Asn Ala Lys Ala
            15              20              25

Gly Leu Cys Gln Thr Phe Val Tyr Gly Gly Cys Arg Ala Lys Glu Asn
        30              35              40

Asn Phe Glu Ser Ala Glu Asp Cys Met Arg Thr Cys Gly Gly Ala
        45              50              55
```

(2) INFORMATION FOR SEQ NO:5:

    (i) SEQUENCE CHARACTERSITICS:

        (A) LENGTH: 418 base pairs
        (B) TYPE: nucleic acid
        (C) STRANDEDNESS: single
        (D) TOPOLOGY: linear

    (ii) MOLECULE TYPE: cDNA

    (vi) ORIGINAL SOURCE:

        (A) ORGANISM: synthetic

    (ix) FEATURE:

        (A) NAME:KEY: CDS
        (B) LOCATION: 77..409

    (ix) FEATURE:

        (A) NAME/KEY: sig_peptide
        (B) LOCATION: 77..235

    (ix) FEATURE:

        (A) NAME/KEY: mat_peptide
        (B) LOCATION: 236..409

    (xi) SEQUENCE DESCRIPTION: SEQ ID NO:5:

GAATTCCATT CAAGAATAGT TCAAACAAGA AGATTACAAA CTATCAATTT CATACACAAT　　60

ATAAACGACC AAAAGA ATG AAG GCT GTT TTC TTG GTT TTG TCC TTG ATC　　109
　　　　　　　Met Lys Ala Val Phe Leu Val Leu Ser Leu Ile
　　　　　　 -53　　　　 -50　　　　　　　　　 -45

GGA TTC TGC TGG GCC CAA CCA GTC ACT GGC GAT GAA TCA TCT GTT GAG　　157
Gly Phe Cys Trp Ala Gln Pro Val Thr Gly Asp Glu Ser Ser Val Glu
　　 -40　　　　　　 -35　　　　　　　　 -30

ATT CCG GAA GAG TCT CTG ATC ATC GCT GAA AAC ACC ACT TTG GCT AAC　　205
Ile Pro Glu Glu Ser Leu Ile Ile Ala Glu Asn Thr Thr Leu Ala Asn
　　 -25　　　　　　 -20　　　　　　　　 -15

GTC GCC ATG GCT GAG AGA TTG GAG AAG AGA AGG CCT GAT TTC TGT TTG　　253
Val Ala Met Ala Glu Arg Leu Glu Lys Arg Arg Pro Asp Phe Cys Leu
-10　　　　　　　 -5　　　　　　　　 1　　　　　　　　 5

GAA CCT CCA TAC ACT GGT CCA TGT AAA GCT AGA ATC ATC AGA TAC TTC　　301
Glu Pro Pro Tyr Thr Gly Pro Cys Lys Ala Arg Ile Ile Arg Tyr Phe
　　　　　　　 10　　　　　　　 15　　　　　　　 20

TAC AAC GCC AAG GCT GGT TTG TGT CAA ACT TTC GTT TAC GGT GGC TGC　　349
Tyr Asn Ala Lys Ala Gly Leu Cys Gln Thr Phe Val Tyr Gly Gly Cys
　　　　　 25　　　　　　　　 30　　　　　　　 35

AGA GCT AAG GAA AAC AAC TTC GAA TCT GCT GAA GAC TGC ATG AGA ACT　　397
Arg Ala Lys Glu Asn Asn Phe Glu Ser Ala Glu Asp Cys Met Arg Thr
　　　　 40　　　　　　　　 45　　　　　　　 50

TGT GGT GGT GCC TAATCTAGA　　418
Cys Gly Gly Ala
　　 55

(2) INFORMATION FOR SEQ ID NO:6:

　　(i) SEQUENCE CHARACTERISTICS:

　　　　(A) KENGTH: 111 amino acids
　　　　(B) TYPE: amino acid
　　　　(D) TOPOLOGY: linear

　　(ii) MOLECULE TYPE: protein

　　(xi) SEQUENCE DESCRIPTION: SEQ ID NO:6:

```
Met Lys Ala Val Phe Leu Val Leu Ser Leu Ile Gly Phe Cys Trp Ala
-53         -50             -45             -40

Gln Pro Val Thr Gly Asp Glu Ser Ser Val Glu Ile Pro Glu Glu Ser
        -35             -30             -25

Leu Ile Ile Ala Glu Asn Thr Thr Leu Ala Asn Val Ala Met Ala Glu
        -20             -15             -10

Arg Leu Glu Lys Arg Arg Pro Asp Phe Cys Leu Glu Pro Pro Tyr Thr
-5              1               5                   10

Gly Pro Cys Lys Ala Arg Ile Ile Arg Tyr Phe Tyr Asn Ala Lys Ala
        15              20                  25
```

(2) INFORMATION FOR SEQ NO:7:

   (i) SEQUENCE CHARACTERSITICS:

      (A) LENGTH: 418 base pairs
      (B) TYPE: nucleic acid
      (C) STRANDEDNESS: single
      (D) TOPOLOGY: linear

   (ii) MOLECULE TYPE: cDNA

   (vi) ORIGINAL SOURCE:

      (A) ORGANISM: synthetic

   (ix) FEATURE:

      (A) NAME:KEY: CDS
      (B) LOCATION: 77..409

   (ix) FEATURE:

      (A) NAME/KEY: sig_peptide
      (B) LOCATION: 77..235

   (ix) FEATURE:

      (A) NAME/KEY: mat_peptide
      (B) LOCATION: 236..409

   (xi) SEQUENCE DESCRIPTION: SEQ ID NO:7:

GAATTCCATT CAAGAATAGT TCAAACAAGA AGATTACAAA CTATCAATTT CATACACAAT     60

ATAAACGACC AAAAGA ATG AAG GCT GTT TTC TTG GTT TTG TCC TTG ATC     109
              Met Lys Ala Val Phe Leu Val Leu Ser Leu Ile
              -53         -50            -45

GGA TTC TGC TGG GCC CAA CCA GTC ACT GGC GAT GAA TCA TCT GTT GAG     157
Gly Phe Cys Trp Ala Gln Pro Val Thr Gly Asp Glu Ser Ser Val Glu
      -40           -35           -30

ATT CCG GAA GAG TCT CTG ATC ATC GCT GAA AAC ACC ACT TTG GCT AAC     205
Ile Pro Glu Glu Ser Leu Ile Ile Ala Glu Asn Thr Thr Leu Ala Asn
      -25           -20           -15

GTC GCC ATG GCT GAG AGA TTG GAG AAG AGA AGG CCT GAT TTC TGT TTG     253
Val Ala Met Ala Glu Arg Leu Glu Lys Arg Arg Pro Asp Phe Cys Leu
-10           -5           1           5

GAA CCT CCA TCT ACT GGT CCA TGT AAA GCT AGA ATC ATC AGA TAC TTC     301
Glu Pro Pro Ser Thr Gly Pro Cys Lys Ala Arg Ile Ile Arg Tyr Phe
        10           15           20

TAC GAC GCC ACT GCT GGT TTG TGT GAA ACT TTC GTT TAC GGT GGC TGC     349
Tyr Asp Ala Thr Ala Gly Leu Cys Glu Thr Phe Val Tyr Gly Gly Cys
        25           30           35

AGA GCT AAC AGA AAC AAC TTC AAG TCT GCT GAA GAC TGC ATG GAA ACT     397
Arg Ala Asn Arg Asn Asn Phe Lys Ser Ala Glu Asp Cys Met Glu Thr
      40           45           50

TGT GGT GGT GCC TAATCTAGA     418
Cys Gly Gly Ala
      55

(2) INFORMATION FOR SEQ ID NO:8:

    (i) SEQUENCE CHARACTERISTICS:

        (A) LENGTH: 111 amino acids
        (B) TYPE: amino acid
        (C) TOPOLOGY: linear

    (ii) MOLECULE TYPE: protein

    (xi) SEQUENCE DESCRIPTION: SEQ ID NO:8:

```
Met Lys Ala Val Phe Leu Val Leu Ser Leu Ile Gly Phe Cys Trp Ala
-53          -50              -45              -40

Gln Pro Val Thr Gly Asp Glu Ser Ser Val Glu Ile Pro Glu Glu Ser
        -35              -30              -25

Leu Ile Ile Ala Glu Asn Thr Thr Leu Ala Asn Val Ala Met Ala Glu
        -20              -15              -10

Arg Leu Glu Lys Arg Arg Pro Asp Phe Cys Leu Glu Pro Pro Ser Thr
-5                  1              5                  10

Gly Pro Cys Lys Ala Arg Ile Ile Arg Tyr Phe Tyr Asp Ala Thr Ala
            15              20              25

Gly Leu Cys Glu Thr Phe Val Tyr Gly Gly Cys Arg Ala Asn Arg Asn
        30              35              40

Asn Phe Lys Ser Ala Glu Asp Cys Met Glu Thr Cys Gly Gly Ala
        45              50              55
```

(2) INFORMATION FOR SEQ ID NO:9:

    (i) SEQUENCE CHARACTERISTICS:

        (A) LENGTH: 418 base pairs
        (B) TYPE: nucleic acid
        (C) STRANDEDNESS: single
        (D) TOPOLOGY: linear

    (ii) MOLECULE TYPE: cDNA

    (vi) ORIGINAL SOURCE:

        (A) ORGANISM: synthetic

    (ix) FEATURE:

        (A) NAME/KEY: CDS
        (B) LOCATION: 77..409

    (ix) FEATURE:

        (A) NAME/KEY: sig_peptide
        (B) LOCATION: 77..235

    (ix) FEATURE:

        (A) NAME/KEY: mat_peptide
        (B) LOCATION: 236..409

    (xi) SEQUENCE DESCRIPTION: SEQ ID NO:9:

GAATTCCATT CAAGAATAGT TCAAACAAGA AGATTACAAA CTATCAATTT CATACACAAT          60

ATAAACGACC AAAAGA ATG AAG GCT GTT TTC TTG GTT TTG TCC TTG ATC          109
                   Met Lys Ala Val Phe Leu Val Leu Ser Leu Ile
                   -53          -50                    -45

GGA TTC TGC TGG GCC CAA CCA GTC ACT GGC GAT GAA TCA TCT GTT GAG          157
Gly Phe Cys Trp Ala Gln Pro Val Thr Gly Asp Glu Ser Ser Val Glu
        -40              -35                    -30

ATT CCG GAA GAG TCT CTG ATC ATC GCT GAA AAC ACC ACT TTG GCT AAC          205
Ile Pro Glu Glu Ser Leu Ile Ile Ala Glu Asn Thr Thr Leu Ala Asn
    -25              -20                    -15

GTC GCC ATG GCT GAG AGA TTG GAG AAG AGA AGG CCT GAT TTC TGT TTG          253
Val Ala Met Ala Glu Arg Leu Glu Lys Arg Arg Pro Asp Phe Cys Leu
-10              -5                     1                5

GAA CCT CCA TCT ACT GGT CCA TGT AAA GCT AGA ATC ATC TTG TAC TTC          301
Glu Pro Pro Ser Thr Gly Pro Cys Lys Ala Arg Ile Ile Leu Tyr Phe
            10                   15                   20

TAC AAC GCC AAG GCT GGT TTG TGT CAA ACT TTC GTT TAC GGT GGC TGC          349
Tyr Asn Ala Lys Ala Gly Leu Cys Gln Thr Phe Val Tyr Gly Gly Cys
        25                   30                   35

AGA GGT AAC GGT AAC CAA TTC TAC TCT GCT GAA GAC TGC ATG AGA ACT          397
Arg Gly Asn Gly Asn Gln Phe Tyr Ser Ala Glu Asp Cys Met Arg Thr
        40                   45                   50

TGT GGT GGT GCC TAATCTAGA          418
Cys Gly Gly Ala
        55

(2) INFORMATION FOR SEQ ID NO:10:

   (i) SEQUENCE CHARACTERISTICS:

      (A) LENGTH: 111 amino acids
      (B) TYPE: amino acid
      (D) TOPOLOGY: linear

   (ii) MOLECULE TYPE: protein

   (xi) SEQUENCE DESCRIPTION: SEQ ID NO:10:

```
Met Lys Ala Val Phe Leu Val Leu Ser Leu Ile Gly Phe Cys Trp Ala
-53          -50              -45              -40

Gln Pro Val Thr Gly Asp Glu Ser Ser Val Glu Ile Pro Glu Glu Ser
        -35              -30              -25

Leu Ile Ile Ala Glu Asn Thr Thr Leu Ala Asn Val Ala Met Ala Glu
        -20              -15              -10

Arg Leu Glu Lys Arg Arg Pro Asp Phe Cys Leu Glu Pro Pro Ser Thr
    -5               1               5               10

Gly Pro Cys Lys Ala Arg Ile Ile Leu Tyr Phe Tyr Asn Ala Lys Ala
            15              20              25

Gly Leu Cys Gln Thr Phe Val Tyr Gly Gly Cys Arg Gly Asn Gly Asn
        30              35              40

Gln Phe Tyr Ser Ala Glu Asp Cys Met Arg Thr Cys Gly Gly Ala
        45              50              55
```

(2) INFORMATION FOR SEQ ID NO:11:

   (i) SEQUENCE CHARACTERISTICS:

      (A) LENGTH: 508 base pairs
      (B) TYPE: nucleic acid
      (C) STRANDEDNESS: single
      (D) TOPOLOGY: linear

   (ii) MOLECULE TYPE: cDNA

   (vi) ORIGINAL SOURCE:

      (A) ORGANISM: synthetic

   (ix) FEATURE:

      (A) NAME/KEY: CDS
      (B) LOCATION: 77..499

   (ix) FEATURE:

      (A) NAME/KEY: sig_peptide
      (B) LOCATION: 77..331

   (ix) FEATURE:

      (A) NAME/KEY: mat_peptide
      (B) LOCATION: 332..499

   (xi) SEQUENCE DESCRIPTION: SEQ ID NO:11:

GAATTCCATT CAAGAATAGT TCAAACAAGA AGATTACAAA CTATCAATTT CATACACAAT          60

ATAAACGATT AAAAGA ATG AGA TTT CCT TCA ATT TTT ACT GCA GTT TTA            109
              Met Arg Phe Pro Ser Ile Phe Thr Ala Val Leu
              -85                 -80                 -75

TTC GCA GCA TCC TCC GCA TTA GCT GCT CCA GTC AAC ACT ACA ACA GAA          157
Phe Ala Ala Ser Ser Ala Leu Ala Ala Pro Val Asn Thr Thr Thr Glu
              -70                 -65                 -60

GAT GAA ACG GCA CAA ATT CCG GCT GAA GCT GTC ATC GGT TAC TCA GAT          205
Asp Glu Thr Ala Gln Ile Pro Ala Glu Ala Val Ile Gly Tyr Ser Asp
              -55                 -50                 -45

TTA GAA GGG GAT TTC GAT GTT GCT GTT TTG CCA TTT TCC AAC AGC ACA          253
Leu Glu Gly Asp Phe Asp Val Ala Val Leu Pro Phe Ser Asn Ser Thr
          -40                 -35                 -30

AAT AAC GGG TTA TTG TTT ATA AAT ACT ACT ATT GCC AGC ATT GCT GCT          301
Asn Asn Gly Leu Leu Phe Ile Asn Thr Thr Ile Ala Ser Ile Ala Ala
          -25                 -20                 -15

AAA GAA GAA GGG GTA TCT TTG GAT AAA AGA GAT TTC TGT TTG GAA CCT          349
Lys Glu Glu Gly Val Ser Leu Asp Lys Arg Asp Phe Cys Leu Glu Pro
-10                 -5                  1                   5

CCA TAC ACT GGT CCA TGT AAA GCT AGA ATC ATC AGA TAC TTC TAC AAC          397
Pro Tyr Thr Gly Pro Cys Lys Ala Arg Ile Ile Arg Tyr Phe Tyr Asn
              10                  15                  20

GCC AAG GCT GGT TTG TGT CAA ACT TTC GTT TAC GGT GGC TGC AGA GCT          445
Ala Lys Ala Gly Leu Cys Gln Thr Phe Val Tyr Gly Gly Cys Arg Ala
          25                  30                  35

AAG TCC AAC AAC TTC GAA TCT GCT GAA GAC TGC ATG AGA ACT TGT GGT          493
Lys Ser Asn Asn Phe Glu Ser Ala Glu Asp Cys Met Arg Thr Cys Gly
          40                  45                  50

GGT GCC TAATCTAGA                                                        508
Gly Ala
    55


(2) INFORMATION FOR SEQ ID NO:12:

   (i) SEQUENCE CHARACTERISTICS:

      (A) LENGTH: 141 amino acids
      (B) TYPE: amino acid
      (C) TOPOLOGY: linear

   (ii) MOLECULE TYPE: protein

   (xi) SEQUENCE DESCRIPTION: SEQ ID NO:12:

```
Met Arg Phe Pro Ser Ile Phe Thr Ala Val Leu Phe Ala Ala Ser Ser
-85             -80             -75             -70

Ala Leu Ala Ala Pro Val Asn Thr Thr Thr Glu Asp Glu Thr Ala Gln
            -65             -60             -55

Ile Pro Ala Glu Ala Val Ile Gly Tyr Ser Asp Leu Glu Gly Asp Phe
            -50             -45             -40

Asp Val Ala Val Leu Pro Phe Ser Asn Ser Thr Asn Asn Gly Leu Leu
            -35             -30             -25

Phe Ile Asn Thr Thr Ile Ala Ser Ile Ala Ala Lys Glu Glu Gly Val
            -20             -15             -10

Ser Leu Asp Lys Arg Asp Phe Cys Leu Glu Pro Pro Tyr Thr Gly Pro
     -5              1               5               10

Cys Lys Ala Arg Ile Ile Arg Tyr Phe Tyr Asn Ala Lys Ala Gly Leu
            15              20              25

Cys Gln Thr Phe Val Tyr Gly Gly Cys Arg Ala Lys Ser Asn Asn Phe
            30              35              40

Glu Ser Ala Glu Asp Cys Met Arg Thr Cys Gly Gly Ala
     45              50              55
```

(2) INFORMATION FOR SEQ ID NO:13:

    (i) SEQUENCE CHARACTERSITICS:

        (A) LENGTH: 508 base pairs
        (B) TYPE: nucleic acid
        (C) STRANDEDNESS: single
        (D) TOPOLOGY: linear

    (ii) MOLECULE TYPE: cDNA

    (vi) ORIGINAL SOURCE:

        (A) ORGANISM: synthetic

    (ix) FEATURE:

        (A) NAME/KEY: CDS
        (B) LOCATION: 77..499

    (ix) FEATURE:

        (A) NAME/KEY: sig_peptide
        (B) LOCATION: 77..331

    (ix) FEATURE:

        (A) NAME/KEY: mat_peptide
        (B) LOCATION: 332..499

(xi) SEQUENCE DESCRIPTION: SEQ ID NO:13:

```
GAATTCCATT CAAGAATAGT TCAAACAAGA AGATTACAAA CTATCAATTT CATACACAAT          60

ATAAACGATT AAAAGA ATG AGA TTT CCT TCA ATT TTT ACT GCA GTT TTA             109
               Met Arg Phe Pro Ser Ile Phe Thr Ala Val Leu
               -85              -80              -75

TTC GCA GCA TCC TCC GCA TTA GCT GCT CCA GTC AAC ACT ACA ACA GAA          157
Phe Ala Ala Ser Ser Ala Leu Ala Ala Pro Val Asn Thr Thr Thr Glu
            -70              -65              -60

GAT GAA ACG GCA CAA ATT CCG GCT GAA GCT GTC ATC GGT TAC TCA GAT          205
Asp Glu Thr Ala Gln Ile Pro Ala Glu Ala Val Ile Gly Tyr Ser Asp
            -55              -50              -45

TTA GAA GGG GAT TTC GAT GTT GCT GTT TTG CCA TTT TCC AAC AGC ACA          253
Leu Glu Gly Asp Phe Asp Val Ala Val Leu Pro Phe Ser Asn Ser Thr
            -40              -35              -30

AAT AAC GGG TTA TTG TTT ATA AAT ACT ACT ATT GCC AGC ATT GCT GCT          301
Asn Asn Gly Leu Leu Phe Ile Asn Thr Thr Ile Ala Ser Ile Ala Ala
            -25              -20              -15

AAA GAA GAA GGG GTA TCT TTG GAT AAA AGA GAT TTC TGT TTG GAA CCT          349
Lys Glu Glu Gly Val Ser Leu Asp Lys Arg Asp Phe Cys Leu Glu Pro
-10                   -5                   1                   5

CCA TAC ACT GGT CCA TGT AAA GCT AGA ATC ATC AGA TAC TTC TAC AAC          397
Pro Tyr Thr Gly Pro Cys Lys Ala Arg Ile Ile Arg Tyr Phe Tyr Asn
               10                   15                   20

GCC AAG GCT GGT TTG TGT CAA ACT TTC GTT TAC GGT GGC TGC AGA GCT          445
Ala Lys Ala Gly Leu Cys Gln Thr Phe Val Tyr Gly Gly Cys Arg Ala
            25                   30                   35

AAG TCC AAC AAC TTC GCT TCT GCT GAA GAC TGC ATG AGA ACT TGT GGT          493
Lys Ser Asn Asn Phe Ala Ser Ala Glu Asp Cys Met Arg Thr Cys Gly
         40                   45                   50

GGT GCC TAATCTAGA                                                         508
Gly Ala
   55
```

(2) INFORMATION FOR SEQ ID NO:14:

(i) SEQUENCE CHARACTERISTICS:

(A) LENGTH: 141 amino acids
(B) TYPE: amino acid
(C) TOPOLOGY: linear

(ii) MOLECULE TYPE: protein

(xi) SEQUENCE DESCRIPTION: SEQ ID NO:14:

```
Met Arg Phe Pro Ser Ile Phe Thr Ala Val Leu Phe Ala Ala Ser Ser
-85             -80             -75                     -70

Ala Leu Ala Ala Pro Val Asn Thr Thr Thr Glu Asp Glu Thr Ala Gln
                -65             -60                 -55

Ile Pro Ala Glu Ala Val Ile Gly Tyr Ser Asp Leu Glu Gly Asp Phe
            -50             -45                 -40

Asp Val Ala Val Leu Pro Phe Ser Asn Ser Thr Asn Asn Gly Leu Leu
        -35             -30             -25

Phe Ile Asn Thr Thr Ile Ala Ser Ile Ala Ala Lys Glu Glu Gly Val
    -20             -15             -10

Ser Leu Asp Lys Arg Asp Phe Cys Leu Glu Pro Pro Tyr Thr Gly Pro
 -5              1           5                   10

Cys Lys Ala Arg Ile Ile Arg Tyr Phe Tyr Asn Ala Lys Ala Gly Leu
            15              20              25

Cys Gln Thr Phe Val Tyr Gly Gly Cys Arg Ala Lys Ser Asn Asn Phe
        30              35              40

Ala Ser Ala Glu Asp Cys Met Arg Thr Cys Gly Gly Ala
    45              50              55
```

(2) INFORMATION FOR SEQ ID NO:15:

(i) SEQUENCE CHARACTERISTICS:

(A) LENGTH: 412 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: syntheric

(ix) FEATURE:

(A) NAME/KEY: CDS
(B) LOCATION: 77..403

(ix) FEATURE:

(A) NAME/KEY: sig_peptide
(B) LOCATION: 77..235

(ix) FEATURE:

(A) NAME/KEY: mat_peptide
(B) LOCATION: 236..403

(xi) SEQUENCE DESCRIPTION: SEQ ID NO:15:

```
GAATTCCATT CAAGAATAGT TCAAACAAGA AGATTACAAA CTATCAATTT CATACACAAT          60

ATAAACGACC AAAAGA ATG AAG GCT GTT TTC TTG GTT TTG TCC TTG ATC            109
                   Met Lys Ala Val Phe Leu Val Leu Ser Leu Ile
                   -53           -50                   -45

GGA TTC TGC TGG GCC CAA CCA GTC ACT GGC GAT GAA TCA TCT GTT GAG          157
Gly Phe Cys Trp Ala Gln Pro Val Thr Gly Asp Glu Ser Ser Val Glu
        -40               -35                   -30

ATT CCG GAA GAG TCT CTG ATC ATC GCT GAA AAC ACC ACT TTG GCT AAC          205
Ile Pro Glu Glu Ser Leu Ile Ile Ala Glu Asn Thr Thr Leu Ala Asn
        -25               -20                   -15

GTC GCC ATG GCT GAG AGA TTG GAG AAG AGG GAT TTC TGT TTG GAA CCT          253
Val Ala Met Ala Glu Arg Leu Glu Lys Arg Asp Phe Cys Leu Glu Pro
-10               -5                   1                   5

CCA TCT ACT GGT CCA TGT AAA GCT AGA ATC ATC AGA TAC TTC TAC GAC          301
Pro Ser Thr Gly Pro Cys Lys Ala Arg Ile Ile Arg Tyr Phe Tyr Asp
            10                15                    20

GCC ACT GCT GGT TTG TGT GAA ACT TTC GTT TAC GGT GGC TGC AGA GCT          349
Ala Thr Ala Gly Leu Cys Glu Thr Phe Val Tyr Gly Gly Cys Arg Ala
            25                30                    35

AAC AGA AAC AAC TTC AAG TCT GCT GAA GAC TGC ATG GAA ACT TGT GGT          397
Asn Arg Asn Asn Phe Lys Ser Ala Glu Asp Cys Met Glu Thr Cys Gly
            40                45                    50

GGT GCC TAATCTAGA                                                        412
Gly Ala
    55
```

(2) INFORMATION FOR SEQ ID NO:16:

(i) SEQUENCE CHARACTERISTICS:

(A) LENGTH: 109 amino acids
(B) TYPE: amino acid
(C) TOPOLOGY: linear

(ii) MOLECULE TYPE: protein

(xi) SEQUENCE DESCRIPTION: SEQ ID NO:16:

```
Met Lys Ala Val Phe Leu Val Leu Ser Leu Ile Gly Phe Cys Trp Ala
-53          -50              -45              -40

Gln Pro Val Thr Gly Asp Glu Ser Ser Val Glu Ile Pro Glu Glu Ser
         -35              -30              -25

Leu Ile Ile Ala Glu Asn Thr Thr Leu Ala Asn Val Ala Met Ala Glu
    -20              -15              -10


Arg Leu Glu Lys Arg Asp Phe Cys Leu Glu Pro Pro Ser Thr Gly Pro
 -5              1              5                   10

Cys Lys Ala Arg Ile Ile Arg Tyr Phe Tyr Asp Ala Thr Ala Gly Leu
         15              20              25

Cys Glu Thr Phe Val Tyr Gly Gly Cys Arg Ala Asn Arg Asn Asn Phe
         30              35              40

Lys Ser Ala Glu Asp Cys Met Glu Thr Cys Gly Gly Ala
    45              50              55
```

(2) INFORMATION FOR SEQ ID NO:17:

    (i) SEQUENCE CHARACTERISTICS:

        (A) LENGTH: 508 base pairs
        (B) TYPE: nucleic acid
        (C) STRANDEDNESS: single
        (D) TOPOLOGY: linear

    (ii) MOLECULE TYPE: cDNA

    (vi) ORIGINAL SOURCE:

        (A) ORGANISM: syntheric

    (ix) FEATURE:

        (A) NAME/KEY: CDS
        (B) LOCATION: 77..499

    (ix) FEATURE:

        (A) NAME/KEY: sig_peptide
        (B) LOCATION: 77..331

    (ix) FEATURE:

        (A) NAME/KEY: mat_peptide
        (B) LOCATION: 332..499

    (xi) SEQUENCE DESCRIPTION: SEQ ID NO:17:

```
GAATTCCATT CAAGAATAGT TCAAACAAGA AGATTACAAA CTATCAATTT CATACACAAT          60

ATAAACGATT AAAAGA ATG AGA TTT CCT TCA ATT TTT ACT GCA GTT TTA            109
               Met Arg Phe Pro Ser Ile Phe Thr Ala Val Leu
               -85              -80               -75

TTC GCA GCA TCC TCC GCA TTA GCT GCT CCA GTC AAC ACT ACA ACA GAA         157
Phe Ala Ala Ser Ser Ala Leu Ala Ala Pro Val Asn Thr Thr Thr Glu
            -70              -65               -60

GAT GAA ACG GCA CAA ATT CCG GCT GAA GCT GTC ATC GGT TAC TCA GAT         205
Asp Glu Thr Ala Gln Ile Pro Ala Glu Ala Val Ile Gly Tyr Ser Asp
            -55              -50               -45


TTA GAA GGG GAT TTC GAT GTT GCT GTT TTG CCA TTT TCC AAC AGC ACA         253
Leu Glu Gly Asp Phe Asp Val Ala Val Leu Pro Phe Ser Asn Ser Thr
            -40              -35               -30

AAT AAC GGG TTA TTG TTT ATA AAT ACT ACT ATT GCC AGC ATT GCT GCT         301
Asn Asn Gly Leu Leu Phe Ile Asn Thr Thr Ile Ala Ser Ile Ala Ala
        -25              -20               -15

AAA GAA GAA GGG GTA TCT TTG GAT AAA AGA GAT TTC TGT TTG GAA CCT         349
Lys Glu Glu Gly Val Ser Leu Asp Lys Arg Asp Phe Cys Leu Glu Pro
-10              -5                1                 5

CCA TAC ACT GGT CCA TGT AAA GCT AGA ATC ATC AGA TAC TTC TAC GAC         397
Pro Tyr Thr Gly Pro Cys Lys Ala Arg Ile Ile Arg Tyr Phe Tyr Asp
              10               15                20

GCC ACT GCT GGT TTG TGT GAA ACT TTC GTT TAC GGT GGC TGC AGA GCT         445
Ala Thr Ala Gly Leu Cys Glu Thr Phe Val Tyr Gly Gly Cys Arg Ala
          25               30                35

AAG AGA AAC AAC TTC AAG TCT GCT GAA GAC TGC ATG GAA ACT TGT GGT         493
Lys Arg Asn Asn Phe Lys Ser Ala Glu Asp Cys Met Glu Thr Cys Gly
        40               45                50

GGT GCC TAATCTAGA                                                        508
Gly Ala
    55
```

(2) INFORMATION FOR SEQ ID NO:18:

    (i) SEQUENCE CHARACTERISTICS:

        (A) LENGTH: 141 amino acids
        (B) TYPE: amino acid
        (C) TOPOLOGY: linear

    (ii) MOLECULE TYPE: protein

    (xi) SEQUENCE DESCRIPTION: SEQ ID NO:18:

```
Met Arg Phe Pro Ser Ile Phe Thr Ala Val Leu Phe Ala Ala Ser Ser
-85                 -80             -75                     -70

Ala Leu Ala Ala Pro Val Asn Thr Thr Thr Glu Asp Glu Thr Ala Gln
            -65             -60                 -55

Ile Pro Ala Glu Ala Val Ile Gly Tyr Ser Asp Leu Glu Gly Asp Phe
        -50             -45                 -40

Asp Val Ala Val Leu Pro Phe Ser Asn Ser Thr Asn Asn Gly Leu Leu
        -35             -30                 -25

Phe Ile Asn Thr Thr Ile Ala Ser Ile Ala Ala Lys Glu Glu Gly Val
        -20             -15                 -10


Ser Leu Asp Lys Arg Asp Phe Cys Leu Glu Pro Pro Tyr Thr Gly Pro
    -5                  1               5                   10

Cys Lys Ala Arg Ile Ile Arg Tyr Phe Tyr Asp Ala Thr Ala Gly Leu
            15                  20                  25

Cys Glu Thr Phe Val Tyr Gly Gly Cys Arg Ala Lys Arg Asn Asn Phe
        30                  35                  40

Lys Ser Ala Glu Asp Cys Met Glu Thr Cys Gly Gly Ala
    45                  50                  55
```

(2) INFORMATION FOR SEQ ID NO:19:

(i) SEQUENCE CHARACTERISTICS:

(A) LENGTH: 412 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: syntheric

(ix) FEATURE:

(A) NAME/KEY: CDS
(B) LOCATION: 77..403

(ix) FEATURE:

(A) NAME/KEY: sig_peptide
(B) LOCATION: 77..235

(ix) FEATURE:

(A) NAME/KEY: mat_peptide
(B) LOCATION: 236..403

(xi) SEQUENCE DESCRIPTION: SEQ ID NO:19:

```
GTC GCC ATG GCT GAG AGA TTG GAG AAG AGG GAT TTC TGT TTG GAA CCT          253
Val Ala Met Ala Glu Arg Leu Glu Lys Arg Asp Phe Cys Leu Glu Pro
-10              -5                  1               5

CCA TCT ACT GGT CCA TGT AAA GCT AGA ATC ATC AGA TAC TTC TAC AAC          301
Pro Ser Thr Gly Pro Cys Lys Ala Arg Ile Ile Arg Tyr Phe Tyr Asn
              10              15              20

GCC AAG GCT GGT TTG TGT CAA ACT TTC GTT TAC GGT GGC TGC AGA GGT          349
Ala Lys Ala Gly Leu Cys Gln Thr Phe Val Tyr Gly Gly Cys Arg Gly
          25              30              35

AAC GGC AAC AAC TTC AAG TCT GCT GAA GAC TGC ATG GAA ACT TGT GGT          397
Asn Gly Asn Asn Phe Lys Ser Ala Glu Asp Cys Met Glu Thr Cys Gly
          40              45              50

GGT GCC TAATCTAGA                                                         412
Gly Ala
    55
```

(2) INFORMATION FOR SEQ ID NO:20:

SEQUENCE CHARACTERISTICS:

(A) LENGTH: 109 amino acids
(B) TYPE: amino acid
(D) TOPOLOGY: linear

(ii) MOLECULE TYPE: protein

(xi) SEQUENCE DESCRIPTION: SEQ ID NO:20:

```
Met Lys Ala Val Phe Leu Val Leu Ser Leu Ile Gly Phe Cys Trp Ala
-53              -50              -45              -40

Gln Pro Val Thr Gly Asp Glu Ser Ser Val Glu Ile Pro Glu Glu Ser
          -35              -30              -25

Leu Ile Ile Ala Glu Asn Thr Thr Leu Ala Asn Val Ala Met Ala Glu
          -20              -15              -10

Arg Leu Glu Lys Arg Asp Phe Cys Leu Glu Pro Pro Ser Thr Gly Pro
-5                  1               5               10

Cys Lys Ala Arg Ile Ile Arg Tyr Phe Tyr Asn Ala Lys Ala Gly Leu
              15              20              25

Cys Gln Thr Phe Val Tyr Gly Gly Cys Arg Gly Asn Gly Asn Asn Phe
          30              35              40

Lys Ser Ala Glu Asp Cys Met Glu Thr Cys Gly Gly Ala
          45              50              55
```

(2) INFORMATION FOR SEQ ID NO:21:

    (i) SEQUENCE CHARACTERISTICS:

        (A) LENGTH: 508 base pairs
        (B) TYPE: nucleic acid
        (C) STRANDEDNESS: single
        (D) TOPOLOGY: linear

    (ii) MOLECULE TYPE: cDNA

    (vi) ORIGINAL SOURCE:

        (A) ORGANISM: synthetic

    (ix) FEATURE:

        (A) NAME/KEY: CDS
        (B) LOCATION: 77..499

    (ix) FEATURE:

        (A) NAME/KEY: sig_peptide
        (B) LOCATION: 77..331

    (ix) FEATURE:

        (A) NAME/KEY: mat_peptide
        (B) LOCATION: 332..499

    (xi) SEQUENCE DESCRIPTION: SEQ ID NO:21:

```
GAATTCCATT CAAGAATAGT TCAAACAAGA AGATTACAAA CTATCAATTT CATACACAAT          60

ATAAACGATT AAAAGA ATG AGA TTT CCT TCA ATT TTT ACT GCA GTT TTA             109
              Met Arg Phe Pro Ser Ile Phe Thr Ala Val Leu
              -85             -80                 -75

TTC GCA GCA TCC TCC GCA TTA GCT GCT CCA GTC AAC ACT ACA ACA GAA          157
Phe Ala Ala Ser Ser Ala Leu Ala Ala Pro Val Asn Thr Thr Thr Glu
            -70             -65                 -60

GAT GAA ACG GCA CAA ATT CCG GCT GAA GCT GTC ATC GGT TAC TCA GAT          205
Asp Glu Thr Ala Gln Ile Pro Ala Glu Ala Val Ile Gly Tyr Ser Asp
            -55             -50                 -45

TTA GAA GGG GAT TTC GAT GTT GCT GTT TTG CCA TTT TCC AAC AGC ACA          253
Leu Glu Gly Asp Phe Asp Val Ala Val Leu Pro Phe Ser Asn Ser Thr
            -40             -35                 -30

AAT AAC GGG TTA TTG TTT ATA AAT ACT ACT ATT GCC AGC ATT GCT GCT          301
Asn Asn Gly Leu Leu Phe Ile Asn Thr Thr Ile Ala Ser Ile Ala Ala
            -25             -20                 -15

AAA GAA GAA GGG GTA TCT TTG GAT AAA AGA GAT TTC TGT TTG GAA CCT          349
Lys Glu Glu Gly Val Ser Leu Asp Lys Arg Asp Phe Cys Leu Glu Pro
-10             -5                  1                   5

CCA TAC ACT GGT CCA TGT AAA GCT AGA ATC ATC AGA TAC TTC TAC AAC          397
Pro Tyr Thr Gly Pro Cys Lys Ala Arg Ile Ile Arg Tyr Phe Tyr Asn
            10              15                  20


GCC AAG GCT GGT TTG TGT CAA ACT TTC GTT TAC GGT GGC TGC AGA GCT          445
Ala Lys Ala Gly Leu Cys Gln Thr Phe Val Tyr Gly Gly Cys Arg Ala
            25              30                  35

AAG TCC AAC AAC TTC AAG TCT GCT GAA GAC TGC ATG GAA ACT TGT GGT          493
Lys Ser Asn Asn Phe Lys Ser Ala Glu Asp Cys Met Glu Thr Cys Gly
            40              45                  50

GGT GCC TAATCTAGA                                                        508
Gly Ala
55
```

(2) INFORMATION FOR SEQ ID NO:22:

SEQUENCE CHARACTERISTICS:

(A) LENGTH: 141 amino acids
(B) TYPE: amino acid
(D) TOPOLOGY: linear

(ii) MOLECULE TYPE: protein

(xi) SEQUENCE DESCRIPTION: SEQ ID NO:22:

```
Met Arg Phe Pro Ser Ile Phe Thr Ala Val Leu Phe Ala Ala Ser Ser
-85              -80              -75              -70

Ala Leu Ala Ala Pro Val Asn Thr Thr Thr Glu Asp Glu Thr Ala Gln
             -65              -60              -55

Ile Pro Ala Glu Ala Val Ile Gly Tyr Ser Asp Leu Glu Gly Asp Phe
             -50              -45              -40

Asp Val Ala Val Leu Pro Phe Ser Asn Ser Thr Asn Asn Gly Leu Leu
             -35              -30              -25

Phe Ile Asn Thr Thr Ile Ala Ser Ile Ala Ala Lys Glu Glu Gly Val
     -20              -15              -10

Ser Leu Asp Lys Arg Asp Phe Cys Leu Glu Pro Pro Tyr Thr Gly Pro
 -5               1               5               10

Cys Lys Ala Arg Ile Ile Arg Tyr Phe Tyr Asn Ala Lys Ala Gly Leu
             15               20               25

Cys Gln Thr Phe Val Tyr Gly Gly Cys Arg Ala Lys Ser Asn Asn Phe
         30               35               40

Lys Ser Ala Glu Asp Cys Met Glu Thr Cys Gly Gly Ala
     45               50               55
```

(2) INFORMATION FOR SEQ ID NO:23:

(i) SEQUENCE CHARACTERISTICS:

(A) LENGTH: 508 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: synthetic

(ix) FEATURE:

(A) NAME/KEY: CDS
(B) LOCATION: 77..499

(ix) FEATURE:

(A) NAME/KEY: sig_peptide
(B) LOCATION: 77..331

(ix) FEATURE:

(A) NAME/KEY: mat_peptide
(B) LOCATION: 332..499

(xi) SEQUENCE DESCRIPTION: SEQ ID NO:23:

```
GAATTCCATT CAAGAATAGT TCAAACAAGA AGATTACAAA CTATCAATTT CATACACAAT           60

ATAAACGATT AAAAGA ATG AGA TTT CCT TCA ATT TTT ACT GCA GTT TTA              109
               Met Arg Phe Pro Ser Ile Phe Thr Ala Val Leu
               -85           -80              -75

TTC GCA GCA TCC TCC GCA TTA GCT GCT CCA GTC AAC ACT ACA ACA GAA           157
Phe Ala Ala Ser Ser Ala Leu Ala Ala Pro Val Asn Thr Thr Thr Glu
            -70              -65              -60

GAT GAA ACG GCA CAA ATT CCG GCT GAA GCT GTC ATC GGT TAC TCA GAT           205
Asp Glu Thr Ala Gln Ile Pro Ala Glu Ala Val Ile Gly Tyr Ser Asp
            -55              -50              -45

TTA GAA GGG GAT TTC GAT GTT GCT GTT TTG CCA TTT TCC AAC AGC ACA           253
Leu Glu Gly Asp Phe Asp Val Ala Val Leu Pro Phe Ser Asn Ser Thr
            -40              -35              -30

AAT AAC GGG TTA TTG TTT ATA AAT ACT ACT ATT GCC AGC ATT GCT GCT           301
Asn Asn Gly Leu Leu Phe Ile Asn Thr Thr Ile Ala Ser Ile Ala Ala
            -25              -20              -15

AAA GAA GAA GGG GTA TCT TTG GAT AAA AGA GAT TTC TGT TTG GAA CCT           349
Lys Glu Glu Gly Val Ser Leu Asp Lys Arg Asp Phe Cys Leu Glu Pro
-10                 -5                  1                 5

CCA TAC ACT GGT CCA TGT AAA GCT AGA ATC ATC AGA TAC TTC TAC AAC           397
Pro Tyr Thr Gly Pro Cys Lys Ala Arg Ile Ile Arg Tyr Phe Tyr Asn
              10                  15                  20

GCC AAG GCT GGT TTG TGT CAA ACT TTC GTT TAC GGT GGC TGC AGA GCT           445
Ala Lys Ala Gly Leu Cys Gln Thr Phe Val Tyr Gly Gly Cys Arg Ala
              25                  30                  35


AAG GAA AAC AAC TTC AAG TCT GCT GAA GAC TGC ATG GAA ACT TGT GGT           493
Lys Glu Asn Asn Phe Lys Ser Ala Glu Asp Cys Met Glu Thr Cys Gly
        40                  45                  50

GGT GCC TAATCTAGA                                                          508
Gly Ala
        55
```

(2) INFORMATION FOR SEQ ID NO:24:

SEQUENCE CHARACTERISTICS:

    (A) LENGTH: 141 amino acids
    (B) TYPE: amino acid
    (D) TOPOLOGY: linear

(ii) MOLECULE TYPE: protein

(xi) SEQUENCE DESCRIPTION: SEQ ID NO:24:

```
Met Arg Phe Pro Ser Ile Phe Thr Ala Val Leu Phe Ala Ala Ser Ser
-85              -80              -75                    -70

Ala Leu Ala Ala Pro Val Asn Thr Thr Thr Glu Asp Glu Thr Ala Gln
            -65              -60              -55

Ile Pro Ala Glu Ala Val Ile Gly Tyr Ser Asp Leu Glu Gly Asp Phe
        -50              -45              -40

Asp Val Ala Val Leu Pro Phe Ser Asn Ser Thr Asn Asn Gly Leu Leu
        -35              -30              -25

Phe Ile Asn Thr Thr Ile Ala Ser Ile Ala Ala Lys Glu Glu Gly Val
    -20              -15              -10

Ser Leu Asp Lys Arg Asp Phe Cys Leu Glu Pro Pro Tyr Thr Gly Pro
 -5               1               5                    10

Cys Lys Ala Arg Ile Ile Arg Tyr Phe Tyr Asn Ala Lys Ala Gly Leu
            15               20               25

Cys Gln Thr Phe Val Tyr Gly Gly Cys Arg Ala Lys Glu Asn Asn Phe
        30               35               40

Lys Ser Ala Glu Asp Cys Met Glu Thr Cys Gly Gly Ala
    45               50               55
```

(2) INFORMATION FOR SEQ ID NO:25:

    (i) SEQUENCE CHARACTERISTICS:

        (A) LENGTH: 508 base pairs
        (B) TYPE: nucleic acid
        (C) STRANDEDNESS: single
        (D) TOPOLOGY: linear

    (ii) MOLECULE TYPE: cDNA

    (vi) ORIGINAL SOURCE:

        (A) ORGANISM: synthetic

    (ix) FEATURE:

        (A) NAME/KEY: CDS
        (B) LOCATION: 77..499

    (ix) FEATURE:

        (A) NAME/KEY: sig_peptide
        (B) LOCATION: 77..331

    (ix) FEATURE:

        (A) NAME/KEY: mat_peptide
        (B) LOCATION: 332..499

(ix) SEQUENCE DESCRIPTION: SEQ ID NO:25:

```
GAATTCCATT CAAGAATAGT TCAAACAAGA AGATTACAAA CTATCAATTT CATACACAAT        60

ATAAACGATT AAAAGA ATG AGA TTT CCT TCA ATT TTT ACT GCA GTT TTA          109
              Met Arg Phe Pro Ser Ile Phe Thr Ala Val Leu
              -85             -80             -75

TTC GCA GCA TCC TCC GCA TTA GCT GCT CCA GTC AAC ACT ACA ACA GAA        157
Phe Ala Ala Ser Ser Ala Leu Ala Ala Pro Val Asn Thr Thr Thr Glu
              -70             -65             -60

GAT GAA ACG GCA CAA ATT CCG GCT GAA GCT GTC ATC GGT TAC TCA GAT        205
Asp Glu Thr Ala Gln Ile Pro Ala Glu Ala Val Ile Gly Tyr Ser Asp
              -55             -50             -45

TTA GAA GGG GAT TTC GAT GTT GCT GTT TTG CCA TTT TCC AAC AGC ACA        253
Leu Glu Gly Asp Phe Asp Val Ala Val Leu Pro Phe Ser Asn Ser Thr
              -40             -35             -30

AAT AAC GGG TTA TTG TTT ATA AAT ACT ACT ATT GCC AGC ATT GCT GCT        301
Asn Asn Gly Leu Leu Phe Ile Asn Thr Thr Ile Ala Ser Ile Ala Ala
              -25             -20             -15

AAA GAA GAA GGG GTA TCT TTG GAT AAA AGA GAT TTC TGT TTG GAA CCT        349
Lys Glu Glu Gly Val Ser Leu Asp Lys Arg Asp Phe Cys Leu Glu Pro
-10                  -5                  1                   5

CCA TAC ACT GGT CCA TGT AAA GCT AGA ATC ATC AGA TAC TTC TAC AAC        397
Pro Tyr Thr Gly Pro Cys Lys Ala Arg Ile Ile Arg Tyr Phe Tyr Asn
                   10              15              20

GCC GAA GCT GGT TTG TGT CAA ACT TTC GTT TAC GGT GGC TGC AGA GCT        445
Ala Glu Ala Gly Leu Cys Gln Thr Phe Val Tyr Gly Gly Cys Arg Ala
              25              30              35

AAG TCC AAC AAC TTC AAG TCT GCT GAA GAC TGC ATG GAA ACT TGT GGT        493
Lys Ser Asn Asn Phe Lys Ser Ala Glu Asp Cys Met Glu Thr Cys Gly
              40              45              50


GGT GCC TAATCTAGA                                                       508
Gly Ala
55
```

(2) INFORMATION FOR SEQ ID NO:26:

(i) SEQUENCE CHARACTERISTICS:

(A) LENGTH: 141 amino acids
(B) TYPE: amino acid
(D) TOPOLOGY: linear

(ii) MOLECULE TYPE: protein

(xi) SEQUENCE DECRIPTION: SEQ ID NO:26:

```
Met Arg Phe Pro Ser Ile Phe Thr Ala Val Leu Phe Ala Ala Ser Ser
-85                 -80                 -75                     -70

Ala Leu Ala Ala Pro Val Asn Thr Thr Thr Glu Asp Glu Thr Ala Gln
            -65                 -60                 -55

Ile Pro Ala Glu Ala Val Ile Gly Tyr Ser Asp Leu Glu Gly Asp Phe
        -50                 -45                 -40

Asp Val Ala Val Leu Pro Phe Ser Asn Ser Thr Asn Asn Gly Leu Leu
        -35                 -30                 -25

Phe Ile Asn Thr Thr Ile Ala Ser Ile Ala Ala Lys Glu Glu Gly Val
    -20                 -15                 -10

Ser Leu Asp Lys Arg Asp Phe Cys Leu Glu Pro Pro Tyr Thr Gly Pro
    -5                  1                   5                   10

Cys Lys Ala Arg Ile Ile Arg Tyr Phe Tyr Asn Ala Glu Ala Gly Leu
            15                  20                  25

Cys Gln Thr Phe Val Tyr Gly Gly Cys Arg Ala Lys Ser Asn Asn Phe
            30                  35                  40

Lys Ser Ala Glu Asp Cys Met Glu Thr Cys Gly Gly Ala
        45                  50                  55
```

(2) INFORMATION FOR SEQ ID NO:27:

(i) SEQUENCE CHARACTERISTICS:

(A) LENGTH: 508 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: synthetic

(ix) FEATURE:

(A) NAME/KEY: CDS
(B) LOCATION: 77..499

(ix) FEATURE:

(A) NAME/KEY: sig_peptide
(B) LOCATION: 77..331

(ix) FEATURE:

(A) NAME/KEY: mat_peptide
(B) LOCATION: 332..499

(xi) SEQUENCE DESCRIPTION: SEQ ID NO:27:

```
GAATTCCATT CAAGAATAGT TCAAACAAGA AGATTACAAA CTATCAATTT CATACACAAT        60

ATAAACGATT AAAAGA ATG AGA TTT CCT TCA ATT TTT ACT GCA GTT TTA          109
               Met Arg Phe Pro Ser Ile Phe Thr Ala Val Leu
               -85             -80             -75

TTC GCA GCA TCC TCC GCA TTA GCT GCT CCA GTC AAC ACT ACA ACA GAA        157
Phe Ala Ala Ser Ser Ala Leu Ala Ala Pro Val Asn Thr Thr Thr Glu
             -70             -65             -60

GAT GAA ACG GCA CAA ATT CCG GCT GAA GCT GTC ATC GGT TAC TCA GAT        205
Asp Glu Thr Ala Gln Ile Pro Ala Glu Ala Val Ile Gly Tyr Ser Asp
         -55             -50             -45

TTA GAA GGG GAT TTC GAT GTT GCT GTT TTG CCA TTT TCC AAC AGC ACA        253
Leu Glu Gly Asp Phe Asp Val Ala Val Leu Pro Phe Ser Asn Ser Thr
         -40             -35             -30

AAT AAC GGG TTA TTG TTT ATA AAT ACT ACT ATT GCC AGC ATT GCT GCT        301
Asn Asn Gly Leu Leu Phe Ile Asn Thr Thr Ile Ala Ser Ile Ala Ala
     -25             -20             -15

AAA GAA GAA GGG GTA TCT TTG GAT AAA AGA GAT TTC TGT TTG GAA CCT        349
Lys Glu Glu Gly Val Ser Leu Asp Lys Arg Asp Phe Cys Leu Glu Pro
-10             -5              1               5

CCA TAC ACT GGT CCA TGT AAA GCT AGA ATC ATC AGA TAC TTC TAC AAC        397
Pro Tyr Thr Gly Pro Cys Lys Ala Arg Ile Ile Arg Tyr Phe Tyr Asn
             10              15              20

GCC GAA GCT GGT TTG TGT CAA ACT TTC GTT TAC GGT GGC TGC AGA GCT        445
Ala Glu Ala Gly Leu Cys Gln Thr Phe Val Tyr Gly Gly Cys Arg Ala
         25              30              35

AAG GAA AAC AAC TTC AAG TCT GCT GAA GAC TGC ATG GAA ACT TGT GGT        493
Lys Glu Asn Asn Phe Lys Ser Ala Glu Asp Cys Met Glu Thr Cys Gly
     40              45              50

GGT GCC TAATCTAGA                                                      508
Gly Ala
     55
```

(2) INFORMATION FOR SEQ ID NO:28:

(i) SEQUENCE CHARACTERISTICS:

(A) LENGTH: 141 amino acids
(B) TYPE: amino acid
(C) TOPOLOGY: linear

(ii) MOLECULE TYPE: protein

(xi) SEQUENCE DESCRIPTION: SEQ ID NO:28:

```
Met Arg Phe Pro Ser Ile Phe Thr Ala Val Leu Phe Ala Ala Ser Ser
-85              -80              -75                    -70

Ala Leu Ala Ala Pro Val Asn Thr Thr Thr Glu Asp Glu Thr Ala Gln
            -65              -60                    -55

Ile Pro Ala Glu Ala Val Ile Gly Tyr Ser Asp Leu Glu Gly Asp Phe
        -50              -45              -40

Asp Val Ala Val Leu Pro Phe Ser Asn Ser Thr Asn Asn Gly Leu Leu
        -35              -30              -25

Phe Ile Asn Thr Thr Ile Ala Ser Ile Ala Ala Lys Glu Glu Gly Val
    -20              -15              -10

Ser Leu Asp Lys Arg Asp Phe Cys Leu Glu Pro Pro Tyr Thr Gly Pro
 -5              1              5                    10

Cys Lys Ala Arg Ile Ile Arg Tyr Phe Tyr Asn Ala Glu Ala Gly Leu
            15              20              25

Cys Gln Thr Phe Val Tyr Gly Gly Cys Arg Ala Lys Glu Asn Asn Phe
        30              35              40

Lys Ser Ala Glu Asp Cys Met Glu Thr Cys Gly Gly Ala
    45              50              55
```

(2) INFORMATION FOR SEQ ID NO:29:

(i) SEQUENCE CHARACTERISTICS:

    (A) LENGTH: 58 amino acids
    (B) TYPE: amino acid
    (C) TOPOLOGY: linear

(ii) MOLECULE TYPE: protein

(xi) SEQUENCE DESCRIPTION: SEQ ID NO:29:

```
Arg Pro Asp Phe Cys Leu Glu Pro Pro Tyr Thr Gly Pro Cys Lys Ala
1              5              10                    15
```

```
Arg Ile Ile Arg Tyr Phe Tyr Asn Ala Lys Ala Gly Leu Cys Gln Thr
            20                      25                      30

Phe Val Tyr Gly Gly Cys Arg Ala Lys Arg Asn Asn Phe Lys Ser Ala
            35                      40                      45

Glu Asp Cys Met Arg Thr Cys Gly Gly Ala
        50                      55
```

**Description**

FIELD OF INVENTION

The present invention relates to aprotinin analogues, a method of producing the analogues and their use in the preparation of medicaments.

BACKGROUND OF THE INVENTION

Aprotinin (also known as bovine pancreatic trypsin inhibitor, BPTI) is a basic protein present in several bovine organs and tissues, such as the lymph nodes, pancreas, lungs, parotid gland, spleen and liver. It is a single-chain polypeptide of 58 amino acid residues with the following amino acid sequence

```
Arg Pro Asp Phe Cys Leu Glu Pro Pro Tyr Thr Gly Pro Cys Lys Ala
Arg Ile Ile Arg Tyr Phe Tyr Asn Ala Lys Ala Gly Leu Cys Gln Thr
Phe Val Tyr Gly Gly Cys Arg Ala Lys Arg Asn Asn Phe Lys Ser Ala
Glu Asp Cys Met Arg Thr Cys Gly Gly Ala
```

The amino acid chain is cross-linked by three disulphide bridges formed between Cys(5) and Cys(55), Cys(14) and Cys(38) and Cys(30) and Cys(51), respectively.

The isoelectric point of aprotinin is quite high (approximately pl 10.5). This is mainly caused by a relatively high content of the positively charged amino acids lysine and arginine. The three-dimensional structure of the aprotinin molecule is very compact which makes it highly stable against denaturation at high temperatures, or by acids, alkalis or organic solvents, or against proteolytic degradation (cf. B. Kassell, Meth. Enzym. 19, 1970, pp. 844-852).

Aprotinin is known to inhibit various serine proteases, including trypsin, chymotrypsin, plasmin and kallikrein, and is used therapeutically in the treatment of acute pancreatitis, various states of shock syndrome, hyperfibrinolytic haemorrhage and myocardial infarction (cf., for instance, J.E. Trapnell et al, Brit. J. Surg. 61, 1974, p. 177; J. McMichan et al., Circulatory shock 9, 1982, p. 107; L.M. Auer et al., Acta Neurochir. 49, 1979, p. 207; G. Sher, Am. J. Obstet. Gynecol. 129, 1977, p. 164; and B. Schneider, Artzneim.-Forsch. 26, 1976, p. 1606). Administration of aprotinin in high doses significantly reduces blood loss in connection with cardiac surgery, including cardiopulmonary bypass operations (cf., for instance, B.P. Bidstrup et al., J. Thorac. Cardiovasc. Surg. 97, 1989, pp. 364-372; W. van Oeveren et al., Ann. Thorac. Surg. 44, 1987, pp. 640-645).

Certain aprotinin analogues are known, e.g. from US 4,595,674 disclosing aprotinin analogues and derivatives wherein Lys(15) is replaced with Gly, Ala, Val, Leu, Ile, Met, Arg, L-α-butyric acid, L-norvaline, L-norleucine,, dehydroalanine or L-homoserine. EP 238 993 discloses aprotinin analogues wherein Lys(15) is replaced with Arg, Val, Ile, Leu, Phe, Gly, Ser, Trp, Tyr or Ala, and wherein Met(52) is furthermore replaced with Glu, Val, Leu, Thr or Ser. EP 307 592 discloses aprotinin analogues wherein one or more of the amino acids in position 15, 16, 17, 18, 34, 39 and 52 are replaced with another amino acid residue. WO 89/01968 discloses a method of producing aprotinin or aprotinin analogues in yeast and specifically discloses analogues lacking one or two amino acid residues at the N-terminal end and in which Lys(41) and/or Arg(42) is replaced with another amino acid residue, in particular Ser. EP 339 942 discloses aprotinin analogues wherein one or more of the amino acids in position 1, 2, 12-19, 38, 41 and 42 are deleted or replaced with another amino acid residue. The amino acid substitutions described in these references are mainly found in the protease-binding region of the aprotinin molecule with a view to changing the protease-inhibition profile of aprotinin except for the substitution of Met(52) according to EP 238 993 and the substitution of Lys(41) and/or Arg(42) according to WO 89/01968 which are carried out to facilitate the production of aprotinin in E. coli and yeast, respectively. EP 339 942 discloses aprotinin analogues modified in one or more of positions 12-19 with a view to changing their protease-binding properties. The analogues may additionally lack one or two amino acid residues at the N-terminal end, and Lys(41) and/or Arg(42) may be substituted by another residue.

RATIONALE OF THE INVENTION

It has previously been described that after intravenous injection of native aprotinin in animals or human volunteers, the plasma level of the inhibitor decreases rather quickly owing to distribution in the extracellular fluid and subsequently accumulation in the kidneys (I. Trautschold et al., in K. Heinkel and H. Schön (Eds.): Pathogenese, Diagnostik, Klinik und Therapie der Erkrankungen des Exokrinen Pankreas, Schattauer, Stuttgart, 1964, p. 289; E. Habermann et al.,

Med. Welt 24(29), 1973, pp. 1163-1167; H. Fritz et al., Hoppe-Seylers Z. Physiol. Chem. 350, 1969, pp. 1541-1550; and H. Kaller et al., Eur. J. Drug Metab. Pharmacokin. 2, 1978, pp. 79-85). Following glomerulus filtration, aprotinin is almost quantitatively bound to the brush border membrane of the proximal tubulus cells. Aprotinin is then reabsorbed into micropinocytic vesicles and phagosomes followed by a very slow degradation in phagolysosomes. This type of transport has been suggested to be representative for peptides in general (M. Just and E. Habermann, Navnyn-Schmiedebergs Arch. Pharmacol. 280, 1973, pp. 161-176; M. Just, Navnyn-Schmiedebergs Arch. Pharmacol. 287, 1975, pp. 85-95).

Macroscopic and histopathological examination following administration of aprotinin reveal changes in the renal tissues of rats, rabbits and dogs after repeated injections of relatively high doses of aprotinin (Bayer, Trasylol, Inhibitor of proteinase; E. Glaser et al. in "Verhandlungen der Deutschen Gesellschaft für Innere Medizin, 78. Kongress", Bergmann, München, 1972, pp. 1612-1614). The nephrotoxicity (i.a. appearing in the form of lesions) observed for aprotinin might be ascribable to the accumulation of aprotinin in the proximal tubulus cells of the kidneys. This nephrotoxicity makes aprotinin less suitable for clinical purposes, in particular those requiring administration of large doses of the inhibitor (such as cardiopulmonary bypass operations).

It would therefore be of considerable interest to produce aprotinin analogues with a reduced nephrotoxicity compared to native aprotinin.


SUMMARY OF THE INVENTION

The invention relates to an aprotinin analogue wherein, to provide a reduced positive net charge, at least one positively charged amino acid residue outside the protease-binding site is removed or replaced with a neutral or negatively charged amino acid residue, and/or wherein at least one negatively charged amino acid residue is inserted or added, and/or wherein at least one neutral amino acid residue is replaced with a negatively charged amino acid residue, and/or wherein, to provide reduced thermal stability of the analogue in aqueous solutions at a pH of about 4-10, one or more amino acid residues outside the protease-binding site, that participate in stabilising the analogue, are deleted, or replaced with one or more other amino acid residues, and/or one or more amino acid residues, that destabilise the analogue, are added outside the protease-binding site,
**with the exception** of such aprotinin analogues wherein the amino acid in position 1 or the amino acids in positions 1 and 2 have been deleted; Ile in position 19 is substituted by another naturally occurring amino acid residue; Lys in position 41 is substituted by another naturally occurring amino acid residue; or Arg in position 42 is substituted by Lys or Ser.

In the present context, the term "reduced positive net charge" is understood to include analogues with a lower positive net charge than that of native aprotinin (which has a positive net charge of +6) as well as with no net charge or a negative net charge. It should be noted that the net charge of aprotinin may vary according to pH, and that the terms "positive net charge", "negative net charge", "positively charged" or "negatively charged" are used about the charge of the molecule at a neutral pH.

The term "protease-binding site" is intended to indicate the amino acid residues which are important for protease inhibition, i.e. the amino acid residues which are in intimate contact with the protease by binding to amino acid residues at or close to the active site of the enzyme. These are currently understood to include (and are, in the present context defined as) the amino acid residues in position 12-18 and 34-39 (cf. H. Fritz and G. Wunderer, Artzneim.-Forsch. 33 (1), 1983, p. 484). It is preferred to remove, insert or replace amino acid residues outside the protease-binding site only in order to avoid substantially changing the protease inhibition profile of the analogue of the invention compared to that of native aprotinin.

It has surprisingly been found that aprotinin analogues with a reduced positive net charge have a significantly lower nephrotoxicity than native aprotinin. One reason for the lower nephrotoxicity may be that aprotinin analogues with a lower positive net charge have a reduced binding affinity for the surface of the proximal tubuli (brush border membrane) so that they are excreted to a large extent in the urine. This explanation is consistent with the findings of H. Fritz et al., op. cit. who report that chemically modified aprotinin derivatives (tetramaleoyl- and pentamaleoyl derivatives) which are less basic than native aprotinin do not bind to an isolated brush border fraction, but are excreted quantitatively in urine.

On the other hand, the chemically modified aprotinin differs greatly from aprotinin and other native proteins in that it contains derivatives of amino acids which are not found in any naturally occurring macromolecules. One should therefore not discount the possibility that the lack of accumulation in the kidneys of the chemically modified derivatives may be ascribed to changed properties of the modified derivatives other than the reduced positive net charge.

Other peptides have been found to bind to the brush border with less affinity and capacity than aprotinin in spite of containing positively charged amino acids and/or having a positive net charge. This indicates that the positive net charge of aprotinin is not the only explanation for the binding and accumulation of aprotinin in the proximal tubulus cells (M. Just et al., 1st. Symp. Physiol., Prop. Pharmacol. Ration.: Kininogenases, Schattauer, Stuttgart 1973, pp.

1163-1167).

Furthermore, it has surprisingly been found that aprotinin analogues with reduced thermal stability do not accumulate in kidney tissue to the same extent as native aprotinin. As described above, the three-dimensional arrangement of aprotinin is very compact which is believed to make the inhibitor highly stable against denaturation and proteolytic degradation. Thus, the accumulation of aprotinin in the kidneys may also be a result of the extraordinary stability of the inhibitor. It is therefore possible that the substitution of one or more amino acid residues in the aprotinin molecule may result in the formation of aprotinin analogues with a reduced stability compared to the native molecule. In the present context, "reduced stability" may, for selection purposes, be expressed as reduced thermal stability of the analogue in aqueous solutions at a pH of about 4-10. The in vivo effect of such reduced stability may be to make the analogue more accessible to degradation, e.g. proteolytic degradation, resulting in faster elimination of the analogue from the proximal tubuli.

It is currently believed that the reduced nephrotoxicity of the analogues of the invention may reside in a combination of a reduced positive net charge and a reduced stability of the molecule.

A contributory cause of damage to the kidneys arising from the administration of native aprotinin may be that aprotinin accumulates on the glomerulus membranes due to affinity for negatively charged structures on the membrane surface. This may result in an enlarged pore size of the glomerulus and consequently an increased permeability to larger molecules, e.g. albumin, which in turn may lead to protein overloading of the kidneys. It is likely that the aprotinin analogue of the invention may have a less damaging effect on the pore size of the glomerulus membrane than native aprotinin due to a decreased affinity for negatively charged structures on that membrane.

Furthermore, it has been observed in some cases that administration of aprotinin leads to an anaphylactoid response. It has been hypothesized that this anaphylactoid response is related to the release of histamine which may have been provoked by the positive net charge of aprotinin. It is therefore envisaged that the anaphylactoid response presumed to be associated with administration of native aprotinin may be reduced or even eliminated by the administration of the aprotinin analogue of the invention.

DETAILED DESCRIPTION OF THE INVENTION

According to the invention, any of the positively charged amino acid residues outside the protease-binding site might be replaced with either the negatively charged amino acid residues Glu or Asp or with any one of the neutral amino acid residues Ala, Cys, Phe, Gly, His, Ile, Leu, Met, Asn, Pro, Glu, Ser, Thr, Val, Trp or Tyr. However, in order to avoid inactive analogues or analogues with an inappropriate three-dimensional structure arising from an undesired folding of the molecule, it is preferred to select substitutions which are identical to amino acid residues in corresponding positions of other protease inhibitors or in domains of larger structures exhibiting a high degree of homology to native aprotinin. In other words, the selection of substituent amino acid residues is preferably based on an analysis of molecules which are homologous to aprotinin. It should be noted that, concomitantly with the amino acid substitution(s) directly contributing to a reduction in the positive net charge, one or more other amino acid substitutions may be carried out which do not in themselves result in a reduced positive net charge, but which may be required in order to produce an active analogue with an appropriate three-dimensional structure.

Accordingly, in a more specific aspect, the present invention relates to an aprotinin analogue which has the general formula I

$R^1$ Asp Phe Cys Leu Glu Pro Pro $R^2$ Thr Gly Pro Cys Lys Ala Arg Ile Ile $R^3$ Tyr Phe Tyr $R^4$ Ala $R^5$ Ala Gly Leu Cys $R^6$ Thr Phe Val Tyr Gly Gly Cys Arg $R^7$ $R^8$ $R^9$ Asn $R^{10}$ Phe $R^{11}$ Ser Ala Glu Asp Cys Met $R^{12}$ Thr Cys Gly Gly Ala

(I)

wherein

$R^1$ is a dipeptide selected from the group consisting of Arg-Pro, Glu-Pro, Asp-Pro, Ala-Pro, Ile-Pro, Thr-Pro, His-Pro, Leu-Pro, Gly-Pro and Ser-Pro, Pro or hydrogen,
$R^2$ is an amino acid residue selected from the group consisting of Tyr, Glu, Asp, Ser, Thr, Ala and Val,
$R^3$ is an amino acid residue selected from the group consisting of Arg, Glu, Asp, Leu, Ser, Ala, Gln and Thr,
$R^4$ is an amino acid residue selected from the group consisting of Asn, Glu and Asp,

4

$R^5$ is an amino acid residue selected from the group consisting of Lys, Glu, Asp, Thr, Val, Ala, Ser, Phe, Gln and Gly,

$R^6$ is an amino acid residue selected from the group consisting of Gln, Glu, Asp, Val and Ala,

$R^7$ is an amino acid residue selected from the group consisting of Ala, Asp, Glu and Gly,

$R^8$ is an amino acid residue selected from the group consisting of Lys, Glu, Asp, Asn, Ser, Thr and Ala,

$R^9$ is an amino acid residue selected from the group consisting of Arg, Glu, Asp, Ser, Asn, Leu, Gly, Gln, Met and Thr,

$R^{10}$ is an amino acid residue selected from the group consisting of Asn, Glu and Asp,

$R^{11}$ is an amino acid residue selected from the group consisting of Lys, Glu, Asp, Leu, Tyr, Ala, Val, Thr, Ser, Pro, His and Ile, and

$R^{12}$ is an amino acid residue selected from the group consisting of Arg, Glu, Asp, Gln, Ala, Asn, His, Gly, Ser and Thr,

with the proviso that at least one of the amino acid residues $R^1$-$R^{12}$ is different from the corresponding amino acid residue of native aprotinin and that when $R^1$ is hydrogen, at least one of the amino acid residues $R^2$ - $R^{12}$ is different from the corresponding amino acid residue of native aprotinin, except that when $R^1$ is hydrogen $R^9$ is not Ser.

Apart from these internal substitutions in the aprotinin molecule, it may be possible to add a peptide containing one or more negatively charged amino acid residues (i.e. Glu or Asp) at the N- or C-terminal end of the aprotinin molecule in order to provide the requisite reduction in the positive net charge. It may further be possible, in order to provide a reduction in the stability of the molecule, to add one or more neutral amino acid residues to the N- or C-terminal end of the molecule. Such additions may be done either to the native aprotinin molecule or in addition to other modifications as indicated above.

If it is desired to change the protease-inhibition properties of the aprotinin analogue apart from reducing its nephrotoxicity, it is possible additionally to modify the analogue in the protease-binding site. For instance, it has previously been demonstrated (cf. H.R. Wenzel and H. Tschesche, Angew. Chem. Internat. Ed. 20, 1981, p. 295) that aprotinin (1-58, Val15) exhibits a relatively high selectivity for granulocyte elastase and an inhibitory effect on collagenase, aprotinin (1-58, Ala15) has a weak effect on elastase, and aprotinin (1-58, Gly15) exhibits an outstanding antitrypsin activity and surprisingly also inhibits kallikrein. Furthermore, it may be possible to modify the inhibitory effect of aprotinin concomitantly with reducing the positive net charge by replacing one or more positively charged amino acids in the protease-binding site by neutral or negatively charged amino acid(s).

Thus, the present invention further relates to an aprotinin analogue which, in addition to being modified outside the protease-binding site is modified by substitution of one or more amino acid residues in the protease-binding site, with one or more other amino acid residues, respectively,

**with the exception** of such aprotinin analogues wherein the amino acid in position 1 or the amino acids in positions 1 and 2 have been deleted; Ile in position 19 is substituted by another naturally occurring amino acid residue; Lys in position 41 is substituted by another naturally occurring amino acid residue; or Arg in position 42 is substituted by Lys or Ser; and

wherein Gly in position 12 is substituted by another naturally occurring amino acid residue; Pro in position 13 is substituted by another naturally occurring amino acid residue; Cys in position 14 is substituted by another naturally occurring amino acid residue; Lys in position 15 is substituted by Arg, Val, Thr, Ile, Leu, Phe, Gly, Ser, Met, Trp, Tyr or Ala; Ala in position 16 is Gly; Arg in position 17 is substituted by another naturally occurring amino acid residue; Ile in position 18 is substituted by Leu, Met, Val or Phe; or Cys in position 38 is substituted by another naturally occurring amino acid residue.

In particular, the analogue has the general formula II

$R^1$ Asp Phe Cys Leu Glu Pro Pro $R^2$ Thr Gly Pro Cys $R^{13}$ $R^{14}$ $R^{15}$ $R^{16}$ $R^{17}$ $R^3$ Tyr Phe Tyr $R^4$ Ala $R^5$ Ala Gly Leu Cys $R^6$ Thr Phe $R^{18}$ Tyr $R^{19}$ Gly Cys $R^{20}$ $R^7$ $R^8$ $R^9$ Asn $R^{10}$ Phe $R^{11}$ Ser Ala Glu Asp Cys Met $R^{12}$ Thr Cys Gly Gly Ala

(II)

wherein $R^1$-$R^{12}$ are as defined above,

$R^{13}$ is an amino acid residue selected from the group consisting of Lys, Arg, Glu, Leu, Met, Tyr and Phe,

$R^{14}$ is an amino acid residue selected from the group consisting of Ala and Gly,

$R^{15}$ is an amino acid residue selected from the group consisting of Arg, Ala, Gly, Lys, Leu, Met, Phe, Tyr, Ile and Asn,

$R^{16}$ is an amino acid residue selected from the group consisting of Ile, Met, Leu, Phe, Thr and Glu,

R[17] is an amino acid residue selected from the group consisting of Ile, Leu, Lys, Gln, Glu, Ser, Arg, Thr and Asn,
R[18] is an amino acid residue selected from the group consisting of Val, Thr, Leu, Ser, Tyr, Gln, His, Pro, Phe, Asn, Ile and Lys,
R[19] is an amino acid residue selected from the group consisting of Gly, Thr and Ser, and
R[20] is an amino acid residue selected from the group consisting of Gln, Lys, Met, Asn, Leu, Gly and Glu,

with the proviso that at least one of the amino acid residues R[1]-R[12] and at least one of the amino acid residues R[13]-R[20] are different from the corresponding amino acid residue of native aprotinin and that when R[1] is hydrogen, R[15] is not Ala, R[17] is not Glu, and R[9] is not Ser, and that when R[15] is Ala, R[9] is not Ser.

Examples of currently favoured aprotinin analogues of the general formula (I) are an analogue wherein R[1] is Glu-Pro, R[5] is Glu, R[8] is Glu, R[11] is Glu, and R[2], R[3], R[4], R[6], R[7], R[9], R[10] and R[12] are as in the native aprotinin sequence; or wherein R[1] is GluPro, R[9] is Glu, R[11] is Glu, and R[2], R[3], R[4], R[6], R[7], R[8], R[10] and R[12] are as in the native aprotinin sequence; or wherein R[9] is Glu, R[11] is Glu, and R[1], R[2], R[3], R[4], R[5], R[6], R[7], R[8], R[10] and R[12] are as in the native aprotinin sequence; or wherein R[2] is Ser, R[4] is Asp, R[5] is Thr, R[6] is Glu, R[8] is Asn, R[12] is Glu, and R[1], R[3], R[7], R[9], R[10], and R[11] are as in the native aprotinin sequence; or wherein R[2] is Ser, R[3] is Leu, R[7] is Gly, R[8] is Asn, R[9] is Gly, R[10] is Gln R[11] is Tyr, and R[1], R[4], R[5], R[6], and R[12] are as in the native aprotinin sequence; or wherein R[1] is a peptide bond, R[9] is Ser, R[11] is Glu, and R[2], R[3], R[4], R[5], R[6], R[7], R[8], R[10] and R[12] are as in the native aprotinin sequence; or wherein R[1] is hydrogen R[9] is Ser, R is Ala, and R[2], R[3], R[4], R[5] R[6], R[7], R[8], R[10] and R[12] are as in the native aprotinin sequence; or wherein R[1] is hydrogen , R[2] is Ser, R[4] is Asp, R[5] is Thr, R[6] is Glu, R[8] is Asn, R[12] is Glu, and R[3], R[7], R[9], R[10] and R[11] are as in the native aprotinin sequence; or wherein R[1] is hydrogen, R[4] is Asp, R[5] is Thr, R[6] is Glu, R[12] is Glu, and R[2], R[3], R[7], R[8], R[9], R[10] and R[11] are as in the native aprotinin sequence; or wherein R[1] is hydrogen, R[2] is Ser, R[7] is Gly, R[8] is Asn, R[9] is Gly, R[12] is Glu, and R[3], R[4], R[5] R[6], R[10] and R[11] are as in the native aprotinin sequence; or wherein R[1] is hydrogen, R[9] is Ser, R[12] is Glu, and R[2], R[3], R4, R[5], R[6], R[7], R[8], R[10] and R[11] are as in the native aprotinin sequence; or wherein R[1] is hydrogen, R[9] is Glu, R[12] is Glu, and R[2], R[3], R[4], R[5], R[6], R[7], R[8], R[10] and R[11] are as in the native aprotinin sequence; or wherein R[1] is hydrogen, R[5] is Glu, R[9] is Ser, R[12] is Glu, and R[2], R[3], R[4], R[6], R[7], R[8], R[10] and R[11] are as in the native aprotinin sequence; or wherein R[1] is hydrogen, R[5] is Glu, R[9] is Glu, R[12] is Glu, and R[2], R[3], R4, R[6], R[7], R[8], R[10] and R[11] are as in the native aprotinin sequence.

In another aspect, the invention relates to a DNA construct encoding an aprotinin analogue according to the invention. The DNA construct of the invention may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to 5 the phosphoamidite method, oligonucleotides are synthesized, e.g. in an automatic DNA synthesizer, purified, annealed, ligated and cloned in suitable vectors.

Alternatively, it is possible to use genomic or cDNA coding for native aprotinin (e.g. obtained by screening a genomic or cDNA library using synthetic oligonucleotide probes) and modified at one or more sites corresponding to the site(s) at which it is desired to introduce amino acid substitutions, e.g. by site-directed mutagenesis using synthetic oligonucleotides encoding the desired amino acid sequence for homologous recombination in accordance with well-known procedures.

In a still further aspect, the invention relates to a recombinant expression vector which comprises a DNA construct of the invention. The recombinant expression vector may be any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

In the vector, the DNA sequence encoding the aprotinin analogue of the invention should be operably connected to a suitable promoter sequence. The promoter may be any DNA sequence which shows transcriptional activity in the host cell of choice and may be derived from genes encoding proteins either homologous or heterologous to the host cell. Examples of suitable promoters for directing the transcription of the DNA encoding the aprotinin analogue of the invention in mammalian cells are the SV 40 promoter (Subramani et al., Mol. Cell Biol. 1, 1981, pp. 854-864), the MT-1 (metallothionein gene) promoter (Palmiter et al., Science 222, 1983, pp. 809-814) or the adenovirus 2 major late promoter. Suitable promoters for use in yeast host cells include promoters from yeast glycolytic genes (Hitzeman et al., J. Biol. Chem. 255, 1980, pp. 12073-12080; Alber and Kawasaki, J. Mol. Appl. Gen. 1, 1982, pp. 419-434) or alcohol dehydrogenase genes (Young et al., in Genetic Engineering of Microorganisms for Chemicals (Hollaender et al, eds.), Plenum Press, New York, 1982), or the TPI1 (US 4, 599, 311) or ADH2-4c (Russell et al., Nature 304, 1983, pp. 652-654) promoters. Suitable promoters for use in filamentous fungus host cells are, for instance, the ADH3 promoter (McKnight et al., The EMBO J. 4, 1985, pp. 2093-2099) or the tpiA promoter.

The DNA sequence encoding the aprotinin analogue of the invention may also be operably connected to a suitable terminator, such as the human growth hormone terminator (Palmiter et al., op. cit.) or (for fungal hosts) the TPI1 (Alber and Kawasaki, op. cit.) or ADH3 (McKnight et al., op. cit.) promoters. The vector may further comprise elements such as polyadenylation signals (e.g. from SV 40 or the adenovirus 5 Elb region), transcriptional enhancer sequences (e. g. the SV 40 enhancer) and translational enhancer sequences (e.g. the ones encoding adenovirus VA RNAs).

The recombinant expression vector of the invention may further comprise a DNA sequence enabling the vector to replicate in the host cell in question. An examples of such a sequence (when the host cell is a mammalian cell) is the SV 40 origin of replication, or (when the host cell is a yeast cell) the yeast plasmid 2μ replication genes REP 1-3 and origin of replication. The vector may also comprise a selectable marker, e.g. a gene the product of which complements a defect in the host cell, such as the gene coding for dihydrofolate reductase (DHFR) or one which confers resistance to a drug, e.g. neomycin, hygromycin or methotrexate, or the Schizosaccharomyces pombe TPI gene (described by P.R. Russell, Gene 40, 1985, pp. 125-130.

The procedures used to ligate the DNA sequences coding for the aprotinin analogue of the invention, the promoter and the terminator, respectively, and to insert them into suitable vectors containing the information necessary for replication, are well known to persons skilled in the art (cf., for instance, Sambrook et al., Molecular Cloning: A Laboratory Manual, Cold Spring Harbor, New York, 1989).

The host cell into which the expression vector of the invention is introduced may be any cell which is capable of producing the aprotinin analogue of the invention and is preferably a eukaryotic cell, such as a mammalian, yeast or fungal cell.

The yeast organism used as the host cell according to the invention may be any yeast organism which, on cultivation, produces large quantities of the aprotinin analogue of the invention. Examples of suitable yeast organisms are strains of the yeast species Saccharomyces cerevisiae, Saccharomyces kluyveri, Schizosaccharomyces pombe or Saccharomyces uvarum. The transformation of yest cells may for instance be effected by protoplast formation followed by transformation in a manner known per se.

Examples of suitable mammalian cell lines are the COS (ATCC CRL 1650), BHK (ATCC CRL 1632, ATCC CCL 10) or CHO (ATCC CCL 61) cell lines. Methods of transfecting mammalian cells and expressing DNA sequences introduced in the cells are described in e.g. Kaufman and Sharp, J. Mol. Biol. 159, 1982, pp. 601-621; Southern and Berg, J. Mol. Appl. Genet. 1, 1982, pp. 327-341; Loyter et al., Proc. Natl. Acad. Sci. USA 79, 1982, pp. 422-426; Wigler et al., Cell 14, 1978, p. 725; Corsaro and Pearson, Somatic Cell Genetics 7, 1981, p. 603, Graham and van der Eb, Virology 52, 1973, p. 456; and Neumann et al., EMBO J. 1, 1982, pp. 841-845.

Alternatively, fungal cells may be used as host cells of the invention. Examples of suitable fungal cells are cells of filamentous fungi, e.g. Aspergillus spp. or Neurospora spp., in particular strains of Aspergillus oryzae or Aspergillus niger.

The use of Aspergillus spp. for the expression of proteins is described in, e.g., EP 272 277.

The present invention further relates to a method of producing an aprotinin analogue according to the invention, the method comprising culturing a cell as described above under conditions conducive to the expression of the aprotinin analogue and recovering the resulting analogue from the culture.

The medium used to cultivate the cells may be any conventional medium suitable for growing mammalian cells or yeast organisms, depending on the choice of host cell. The aprotinin analogue will be secreted by the host cells to the growth medium and may be recovered therefrom by conventional procedures including separating the cells from the medium by centrifugation or filtration, precipitating the proteinaceous components of the supernatant or filtrate by means of a salt, e.g. ammonium sulfate, purification by a variety of chromatographic procedures, e.g. ion exchange chromatography or affinity chromatography, or the like.

The present invention also relates to a pharmaceutical composition comprising an aprotinin analogue of the invention together with a pharmaceutically acceptable carrier or excipient. In the composition of the invention, the aprotinin analogue may be formulated by any of the established methods of formulating pharmaceutical compositions, e. g. as described in Remington's Pharmaceutical Sciences, 1985. The composition may typically be in a form suited for systemic injection or infusion and may, as such, be formulated with sterile water or an isotonic saline or glucose solution.

In a yet further aspect, the present invention relates to the use of an aprotinin analogue of the invention for the manufacture of a medicament with reduced nephrotoxicity compared to native aprotinin and/or a medicament which, on administration, gives rise to a reduced incidence of anaphylactoid reactions compared to those experienced with native aprotinin.

As noted above, it has been found that native aprotinin administered in doses approximating clinical doses exhibits a deleterious effect on the kidneys. This effect may arise from the extraordinarily high stability and relatively high positive net charge of the aprotinin molecule. The aprotinin analogue of the invention is therefore contemplated to be advantageous to use for the therapeutic applications suggested for native aprotinin, in particular those which necessitate the use of large aprotinin doses. Therapeutic applications for which the use of the aprotinin analogue of the invention is indicated as a result of its inhibition of human serine proteases, e.g. trypsin, plasmin, kallikrein, elastase and cathepsin G, include (but are not limited to) acute pancreatitis, inflammation, thrombocytopenia, preservation of platelet function, organ preservation, wound healing, shock (including shock lung) and conditions involving hyperfibrinolytic haemorrhage. A high dose of aprotinin is indicated during and after cardiopulmonary bypass operations; the lower nephrotoxicity of the present aprotinin analogue is therefore of particular interest for this application and possibly in other surgery involving (major) loss of blood, as is the possibly reduced risk of causing anaphylactoid response due to the lower positive net charge of the analogue.

BRIEF DESCRIPTION OF THE DRAWINGS

The invention is further described in the following examples with reference to the appended drawings, wherein

**Fig. 1** shows the construction of a synthetic aprotinin gene from oligonucleotide sequences;
**Fig. 2** shows the construction of plasmid pKFN-1503;
**Fig. 3** is a graph showing the inhibitory activity in urine and kidneys after administration of aprotinin analogues with different net charges and thermal stability;
**Fig. 4** shows the inhibitory activity in urine 3 hours after administration of aprotinin analogues with different net charges;
**Fig. 5** shows the inhibitory activity in kidneys 3 hours after administration of aprotinin analogues with a different thermal stability; and
**Fig. 6** shows the accumulation of inhibitory activity in kidneys after administration of aprotinin analogues with different thermal stability. The accumulation index is calculated as the inhibitory activity after 3 hours divided by the inhibitory activity after 1 hour.

The present invention is further illustrated in the following examples which are not intended to be in any way limiting to the scope of the invention as claimed.

Example 1

Production of [Glu1, Glu26, Glu41, Glu46]-aprotinin from yeast strain KFN-1512

A synthetic gene coding for [Glu1, Glu26, Glu41, Glu46]-aprotinin was constructed from 10 oligonucleotides by ligation. The oligonucleotides were synthesized on an automatic DNA synthesizer using phosphoramidite chemistry on a controlled pore glass support (Beaucage, S.L., and Caruthers, M.H., Tetrahedron Letters 22, (1981) 1859-1869). The following 10 oligonucleotides were synthesized:

NOR-1948: CATGGCTGAGAGATTGGAGAAGAGAGAGCCTGATTTCTGTTTGGAAC-CTCCATACACTGGTCC

NOR-1947: TTACATGGACCAGTGTATGGAGGTTCCAAACAGAAATCAG-GCTCTCTCTTCTCCAATCTCTCAGC

NOR-354: ATGTAAAGCTAGAATCATCAGATACTTCTACAACG

NOR-1939: TTCGGCGTTGTAGAAGTATCTGATGATTCTAGCT

NOR-1938: CCGAAGCTGGTTTGTGTCAAACTTTCGTTTACGGTGGCT

NOR-357: CTCTGCAGCCACCGTAAACGAAAGTTTGACACAAACCAGC

NOR-1940: GCAGAGCTGAAAGAAACAACTTCGAAT

NOR-1949: AGCAGATTCGAAGTTGTTTCTTTCAG

NOR-360: CTGCTGAAGACTGCATGAGAACTTGTGGTGGTGCCTAAT

NOR-361: CTAGATTAGGCACCACCACAAGTTCTCATGCAGTCTTC

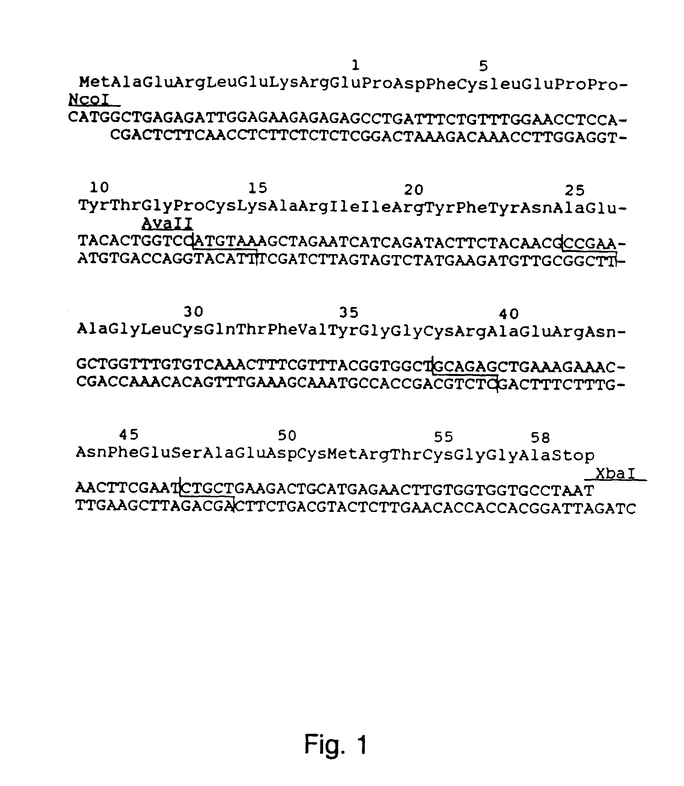

5 duplexes A - E were formed from the above 10 oligonucleotides as shown in Fig. 1.

20 pmole of each of the duplexes A - E were formed from the corresponding pairs of 5'-phosphorylated oligonucleotides by heating for 5 min. at 90°C followed by cooling to room temperature over a period of 75 minutes. The five duplexes were mixed and treated with $T_4$ DNA ligase. The synthetic gene was isolated as a 203 bp band after electrophoresis of the ligation mixture on a 2% agarose gel. The obtained synthetic gene is shown in Fig. 1.

The synthetic gene was ligated to a 209 bp EcoRI-NcoI fragment from pLaC212spx3 and to the 2.8 Kb EcoRI-XbaI fragment of plasmid pTZ19R (Mead, D.A., Szczesna-Skorupa, E. and Kemper, B., Prot. Engin. 1 (1986) 67-74). Plasmid pLaC212spx3 is described in Example 3 of International Patent Application No. PCT/DK88/00147.

The 209 bp EcoRI-NcoI fragment from pLaC212spx3 encodes a synthetic yeast leader peptide.

The ligation mixture was used to transform a competent E. coli strain r⁻, m⁺) selecting for ampicillin resistance. DNA sequencing (Sanger, F., Micklen, S., and Coulson, A.R., Proc. Natl. Acad. Sci. USA 74 (1977) 5463-5467) showed that plasmids from the resulting colonies contained the correct DNA sequence for [Glu1, Glu26, Glu41, Glu46]-aprotinin.

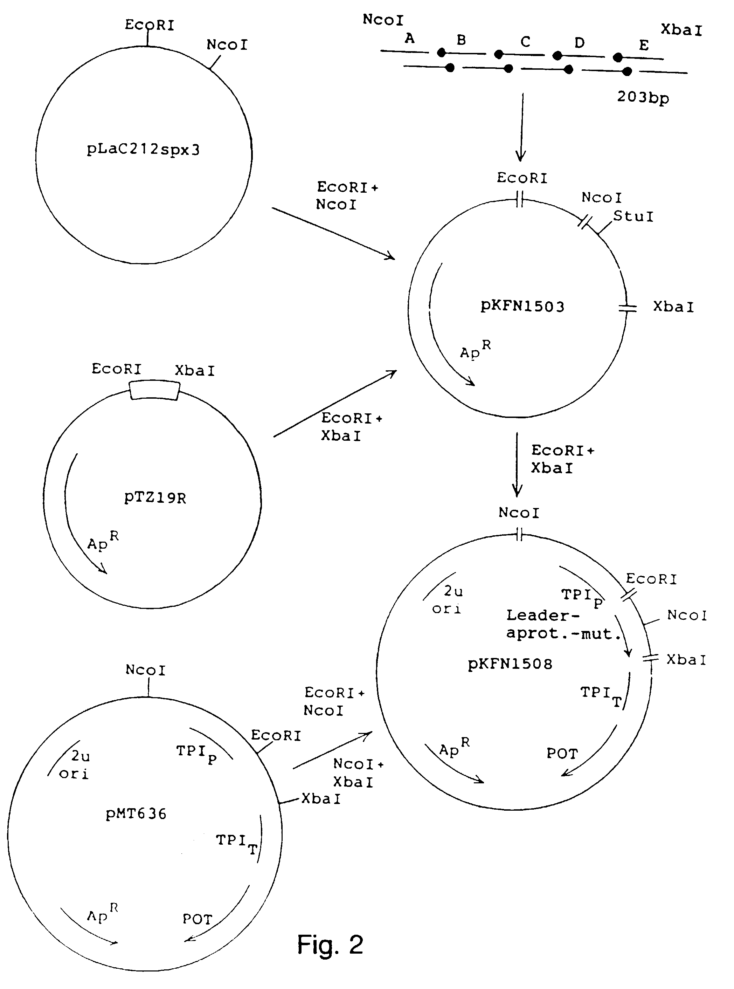

One plasmid pKFN-1503 was selected for further use. The construction of plasmid pKFN-1503 is illustrated in Fig. 2.

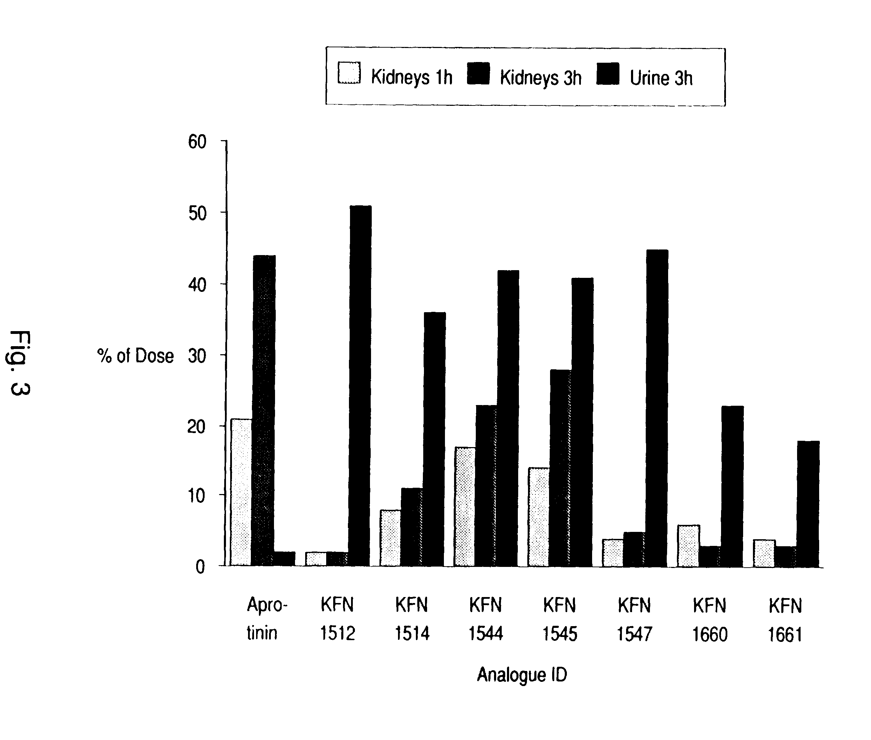

pKFN-1503 was cut with EcoRI and XbaI and the 412 bp fragment was ligated to the 9.5 kb NcoI-XbaI fragment from pMT636 and the 1.4 kb NcoI-EcoRI fragment from pMT636, resulting in plasmid pKFN-1508, see Fig. 3. Plasmid pMT636 is described in International Patent application No. PCT/DK88/00138.

pMT636 is an E. coli - S. cerevisiae shuttle vector containing the Schizosaccharomyces pombe TPI gene (POT) (Russell, P.R., Gene 40 (1985) 125-130), the S. cerevisiae triosephosphate isomerase promoter and terminator, $TPI_p$ and $TPI_T$ (Alber, T., and Kawasaki, G. J. Mol. Appl. Gen. 1 (1982), 419-434). Plasmid pKFN-1508 contains the following sequence:

$TPI_p$-LaC212spx3 signal-leader (1-47)-Glu(ArgLeuGluLysArg [Glu1, Glu26, Glu41, Glu46]-aprotinin-$TPI_T$ where LaC212spx3 signal-leader is the synthetic yeast leader described in International Patent Application No. PCT/DK88/00147. The DNA sequence of the 412 bp EcoRI-XbaI fragment from pKFN-1503 and pKFN-1508 is shown in Sequence Listing ID No. 1.

S. cerevisiae strain MT663 (E2-7B XE11-36 a/α, tpi/ tpi, pep 4-3/pep 4-3) was grown on YPGaL (1% Bacto yeast extract, 2% Bacto peptone, 2% galactose, 1% lactate) to an O.D. at 600 nm of 0.6.

100 ml of culture was harvested by centrifugation, washed with 10 ml of water, recentrifugated and resuspended in 10 ml of a solution containing 1.2 M sorbitol, 25 mM $Na_2$EDTA pH = 8.0 and 6.7 mg/ml dithiotreitol. The suspension was incubated at 30°C for 15 minutes, centrifuged and the cells resuspended in 10 ml of a solution containing 1.2 M sorbitol, 10 mM $Na_2$EDTA, 0.1 M sodium citrate, pH = 5.8, and 2 mg Novozym® 234. The suspension was incubated at 30°C for 30 minutes, the cells collected by centrifugation, washed in 10 ml of 1.2 M sorbitol and 10 ml of CAS (1.2 M sorbitol, 10 mM $CaCl_2$, 10 mM Tris HCl (Tris = Tris(hydroxymethyl)aminomethane) pH = 7.5) and resuspended in 2 ml of CAS. For transformation, 0.1 ml of CAS-resuspended cells were mixed with approx. 1 μg of plasmid pKFN-1508 and left at room temperature for 15 minutes. 1 ml of (20% polyethylene glycol 4000, 20 mM $CaCl_2$, 10 mM $CaCl_2$, 10 mM Tris HCl, pH = 7.5) was added and the mixture left for a further 30 minutes at room temperature. The mixture was centrifuged and the pellet resuspended in 0.1 ml of SOS (1.2 M sorbitol, 33% v/v YPD, 6.7 mM $CaCl_2$, 14 μg/ml leucine) and incubated at 30°C for 2 hours. The suspension was then centrifuged and the pellet resuspended in 0.5 ml of 1.2 M sorbitol. Then, 6 ml of top agar (the SC medium of Sherman et al., (Methods in Yeast Genetics, Cold Spring Harbor Laboratory (1982)) containing 1.2 M sorbitol plus 2.5% agar) at 52°C was added and the suspension poured on top of plates containing the same agar-solidified, sorbitol containing medium.

Transformant colonies were picked after 3 days at 30°C, reisolated and used to start liquid cultures. One such transformant KFN-1512 was selected for further characterization.

Yeast strain KFN-1512 was grown on YPD medium (1% yeast extract, 2% peptone (from Difco Laboratories), and 6% glucose). A 200 ml culture of the strain was shaken at 250 rpm at 30°C for 3 days to an O.D. at 600 nm of about 20. After centrifugation, the supernatant was analyzed by FPLC ion exchange chromatography. The yeast supernatant was filtered through a 0.22 $\mu$m Millex GV filter unit, and 1 ml was applied on a MonoS cation exchange column (0.5 x 5 cm) equilibrated with 20 mM formic acid, pH 3.7. After washing with equilibration buffer, the column was eluted with a linear NaCl gradient (0.1 M) in equilibration buffer. Trypsin inhibitor activity was quantified in the eluted fractions by spectrophotometric assay (Kassel, B., Methods Enzymol. 19 (1970), 844-852) and furthermore by integration of absorption at 280 nm from

$$E_{280}^{1\%} \quad (\text{aprotinin}) = 8.3$$

In order to obtain material for toxicology studies, yeast strain KFN-1512 was grown on a larger scale. The aprotinin analogue was purified by a combination of ion exchange chromatography and reverse phase HPLC.

## Example 2

### Production of [Glu1, Glu42, Glu46] aprotinin from yeast strain KFN-1514

A synthetic gene coding for [Glu1, Glu42, Glu46]-aprotinin was constructed from 10 oligonucleotides by ligation as described in example 1.

The pTZ19R-derived plasmid pKFN-1505 containing the synthetic gene fused in frame to a synthetic yeast leader peptide was constructed as described in Example 1.

By following the procedure of Example 1, a yeast expression plasmid pKFN-1510 was obtained containing the following construction TPI$_p$-LaC212spx3 signal-leader(1-47)-GluArgLeuGluLysArg [Glu1, Glu42, Glu46]-aprotinin-TPI$_T$.

The DNA sequence of the 412 bp EcoRI-XbaI fragment from pKFN-1505 and pKFN-1510 is given in Sequence Listing ID No. 3.

Plasmid pKFN-1510 was transformed in yeast strain MT663 as described above resulting in yeast strain KFN-1514.

Culturing of the transformed strain KFN-1514 in YPD-medium, analysis for [Glu1, Glu42, Glu46]-aprotinin in the supernatant, and production of material for toxicological studies was performed as described above.

## Example 3

### Production of [Glu42, Glu46]-aprotinin from yeast strain KFN-1544

The 144 bp AvaII-XbaI fragment encoding [Glu42, Glu46]-aprotinin (12-58) from pKFN-1505 was used to replace the corresponding DNA fragment encoding aprotinin(12-58) from plasmid pKFN-1000 resulting in plasmid pKFN-1528. Plasmid pKFN-1000 is described in example 4 of International Patent Application, Publication No. WO 90/10075.

By following the procedure of example 1, a yeast expression plasmid pKFN-1541 was obtained containing the following construction TPI$_p$-LaC212spx3 signal-leader(1-47)-GluArgLeuGluLysArg-[Glu42, Glu46]-aprotinin-TPI$_T$.

The DNA sequence of the 412 bp EcoRI-XbaI fragment from pKFN-1528 and pKFN-1541 is given in Sequence Listing ID No. 5.

Plasmid pKFN-1541 was transformed in yeast strain MT663 ad described above resulting in yeast strain KFN-1544.

Culturing of the transformed strain KFN-1544 in YPD-medium, analysis for [Glu42, Glu46]-aprotinin in the supernatant and production of material for toxicological studies was performed as described above.

## Example 4

### Production of [Ser10, Asp24, Thr26, Glu31, Asn41, Glu53]-aprotinin from yeast strain KFN-1545

The synthetic gene coding for [Ser10, Asp24, Thr26, Glu31, Asn41, Glu53] was constructed from 10 oligonucleotides by ligation as described in example 1.

The pTZ19R-derived plasmid pKFN-1530 containing the synthetic gene fused in frame to a synthetic yeast leader peptide sequence was constructed as described in example 1.

By following the procedure of example 1, a yeast expression plasmid pKFN-1532 was obtained containing the following construction TPI$_p$-LaC212spx3 signal-leader(1-47)-GluArgLeuGluLysArg-[Ser10, Asp24, Thr26, Glu31, Asn41, Glu52]-aprotinin-TPI$_T$.

The DNA sequence of the 412 bp EcoRI-XbaI fragment from pKFN-1530 and pKFN-1532 is given in Sequence Listing ID No. 7.

Plasmid pKFN-1532 was transformed in yeast strain MT663 ad described above resulting in yeast strain KFN-1545.

Culturing of the transformed strain KFN-1545 in YPD-medium, analysis for [Ser10, Asp24, Thr26, Glu31, Asn41, Glu53]-aprotinin in the supernatant and production of material for toxicological studies was performed as described above.

## Example 5

### Production of [Ser10, Leu20, Gly40, Asn41, Gln44 Tyr46]-aprotinin from yeast strain KFN-1547

The synthetic gene coding for [Ser10, Leu20, Gly40, Asn41, Gly42, Gln44, Tyr46]-aprotinin was constructed from 10 oligonucleotides by ligation as described in example 1.

The pTZ19R-derived plasmid pKFN-1534 containing the synthetic gene fused in frame to a synthetic yeast leader peptide sequence was constructed as described in example 1.

By following the procedure of example 1, a yeast expression plasmid pKFN-1537 was obtained containing the following construction TPI$_p$-LaC212spx3 signal-leader(1-47) GluArgLeuGluLysArg-[Ser10, Leu20, Gly40, Asn41, Gly42, Gln44, Tyr46]-aprotinin-TPI$_T$.

The DNA sequence of the 412 bp EcoRI-XbaI fragment from pKFN-1534 and pKFN-1537 is given in Sequence Listing ID No. 9.

Plasmid pKFN-1537 was transformed in yeast strain MT663 ad described above resulting in yeast strain KFN-1547.

Culturing of the transformed strain KFN-1547 in YPD-medium, analysis for [Ser10, Leu20, Gly40, Asn41, Gly42, Gln44, Tyr46]-aprotinin in the supernatant and production of material for toxicological studies was performed as described above.

## Example 6

### Production of Des-Arg1, des-Pro2-[Ser 42,Glu46]-aprotinin from yeast strain KFN-1660

The 1.4 kb AhaII-StyI fragment and the 1.8 kb AhaII-SalI fragment both from plasmid pKFN-306 were ligated to a duplex consisting of the following two synthetic oligonucleotides:

```
NOR-2188: 5'  CAAGGCTGGTTTGTGTCAAACTTTCGTTTACGGTGGCTGCA-
              GAGCTAAGTCCAACAACTTCGAATCTGCTGAAGACTGCATG-
              AGAACTTGTGGTGGTGCCTAATCTAGAG  3'


NOR-2189: 5'  TCGACTCTAGATTAGGCACCACCACAAGTTCTCATGCAGTC-
              TTCAGCAGATTCGAAGTTGTTGGACTTAGCTCTGCAGCCAC-
              CGTAAACGAAAGTTTGACACAAACCAGC  3'
```

Plasmid pKFN-306 is a pTZ19R derived plasmid with a 502 bp EcoRI-XbaI insert containing the Saccharomyces cerevisiae mating factor alpha 1 signal-leader (1-85) gene fused in-frame with a synthetic gene for des-Arg1, des-Pro2-[Ser42]-aprotinin. The construction of plasmid pKFN-306 is described in WO 89/01968.

The ligation mixture was used to transform a competent E. coli strain (r⁻, m⁺) selecting for ampicillin resistance. DNA sequencing (Sanger, F., Micklen, S., and Coulsen, A.R., Proc. Natl.Acad.Sci. USA 74 (1977) 5463-5467) showed that plasmids from the resulting colonies contained the correct sequence for des-Arg1, Pro2-[Ser42, Glu46] aprotinin.

One plasmid pKFN-1629 was selected for further use.

By following the procedure of Example 1, a yeast expression plasmid pKFN-1656 was obtained containing the following construction TPI$_p$-MFα1 signal-leader(1-85)-des-Arg1, des-Pro2-[Ser42,Glu46]-aprotinin-TPI$_T$.

The DNA sequence of the 502 bp EcoRI-Xbal fragment from pKFN-1629 and pKFN-1656 is given in Sequence Listing ID No. 11.

Plasmid pKFN-1656 was transformed in yeast strain MT663 as described above resulting in yeast strain KFN-1660.

Culturing of the transformed strain KFN-1660 in YPD-medium, analysis for des-Arg1, des-Pro2-[Ser42, Glu46]-aprotinin in the supernatant and production of material for toxicological studies was performed as described above.

Example 7

Production of Des-Arg1,des-Pro2-[Ser42,Ala46]-aprotinin from yeast strain KFN-1661

The 1.4 kb AhaII-StyI fragment and the 1.8 kb AhaII-SalI fragment both from plasmid pKFN-306 were ligated to a duplex consisting of the following two synthetic oligonucleotides:

NOR-2196: 5'    CAAGGCTGGTTTGTGTCAAACTTTCGTTTACGGTGGCTGCA-
GAGCTAAGTCCAACAACTTCGCTTCTGCTGAAGACTGCATG-
AGAACTTGTGGTGGTGCCTAATCTAGAG 3'

NOR-2197: '5    TCGACTCTAGATTAGGCACCACCACAAGTTCTCATGCAGTC-
TTCAGCAGAAGCGAAGTTGTTGGACTTAGCTCTGCAGCCAC-
CGTAAACGAAAGTTTGACACAAACCAGC   3'

Plasmid pKFN-306 is a pTZ19R derived plasmid with a 502 bp EcoRI-Xbal insert containing the Saccharomyces cerevisiae mating factor alpha 1 signal-leader (1-85) gene fused in-frame with a synthetic gene for des-Arg1, des-Pro2-[Ser42]-aprotinin. The construction of plasmid pKFN-306 is described in WO 89/01968.

The ligation mixture was used to transform a competent E. coli strain (r, m$^+$) selecting for ampicillin resistance. DNA sequencing (Sanger, F., Micklen, S., and Coulsen, A.R., Proc.Natl.Acad.Sci. USA 74 (1977) 5463-5467) showed that plasmids from the resulting colonies contained the correct sequence for des-Arg1, des-Pro2-[Ser42,Glu46] aprotinin.

One plasmid pKFN-1631 was selected for further use.

By following the procedure of Example 1, a yeast expression plasmid pKFN-1657 was obtained containing the following construction TPI$_P$-MF$\alpha$1 signal-leader(1-85)-des-Arg1, des-Pro2-[Ser42,Ala46]-aprotinin-TPI$_T$.

The DNA sequence of the 502 bp EcoRI-Xbal fragment form pKFN-1631 and pKFN-1657 is given in Sequence Listing ID No. 13.

Plasmid pKFN-1657 was transformed in yeast strain MT663 as described above resulting in yeast strain KFN-1661.

Culturing of the transformed strain KFN-1661 in YPD-medium, analysis for des-Arg1, des-Pro2-[Ser42,Ala46]-aprotinin in the supernatant and production of material for toxicological studies was performed as described above.

Example 8

Production of Des-Arg1,des-Pro2-[Ser10, Asp24, Thr26, Glu31, Asn41, Glu53]-aprotinin from yeast strain KFN-1735

A synthetic gene coding for des-Arg1,des-Pro2-[Ser10, Asp24, Thr26, Glu31, Asn41, Glu53]-aprotinin was constructed from 10 oligonucleotides by ligation as described in example 1.

The pTZ19R-derived plasmid pKFN-1707 containing the synthetic gene fused in frame to a synthetic yeast leader peptide was constructed as described in Example 1.

By following the procedure of Example 1, a yeast expression plasmid pKFN-1709 was obtained containing the following construction TPI$_p$-LaC212spx3 signal-leader(1-47)-GluArgLeuGluLysArg-des Arg1,des-Pro2-[Ser10, Asp24, Thr26, Glu31, Asn41, Glu53]-aprotinin-TPI$_T$.

The DNA sequence of the 406 bp EcoRI-Xbal fragment from pKFN-1707 and pKFN-1709 is given in Sequence Listing ID No. 15 .

Plasmid pKFN-1709 was transformed in yeast strain MT663 as described above resulting in yeast strain KFN-1735.

Culturing of the transformed strain KFN-1735 in YPD-medium, analysis for des-Arg1,des-Pro2-[Ser10, Asp24, Thr26, Glu31, Asn41, Glu53]-aprotinin in the supernatant and production of material for toxicological studies was performed as described above.

Example 9

Production of Des-Arg1,des-Pro2-[Asp24,Thr26, Glu31, Glu53]-aprotinin from yeast strain KFN-1737.

The 1.8 kb AhaII-XbaI fragment and the 1.4 kb AhaII-AvaII fragment both from plasmid pKFN-306 ( see example 5) were ligated to a synthetic 141 bp AvaII-XbaI fragment encoding [Asp24, Thr26, Glu31, Glu53]-aprotinin.

The resulting pTZ19R-derived plasmid was pKFN-1711.

By following the procedure of Example 1, a yeast expression plasmid pKFN-1713 was obtained containing the following construction $TPI_p$-MF$\alpha$1 signal-leader(1-85)-des-Arg1,des-Pro2-[Asp24, Thr26, Glu31, Glu53]-aprotinin-$TPI_T$.

The DNA sequence of the 502 bp EcoRI-XbaI fragment from pKFN-1711 and pKFN-1713 is given in Sequence Listing ID No. 17 .

Plasmid pKFN-1713 was transformed in yeast strain MT663 as described above resulting in yeast strain KFN-1737.

Culturing of the transformed strain KFN-1737 in YPD-medium, analysis for des-Arg1,des-Pro2-[Asp24, Thr26, Glu31, Glu53]-aprotinin in the supernatant and production of material for toxicological studies was performed as described above.

Example 10

Production of Des-Arg1,des-Pro2-[Ser10, Gly40, Asn41,Gly42 Glu53]-aprotinin from yeast strain KFN-1739

A synthetic gene coding for des-Arg1,des-Pro2-[Ser10, Gly40, Asn41, Gly42, Glu53]-aprotinin was constructed from 10 oligonucleotides by ligation as described in example 1.

The pTZ19R-derived plasmid pKFN-1715 containing the synthetic gene fused in frame to a synthetic yeast leader peptide was constructed as described in Example 1.

By following the procedure of Example 1, a yeast expression plasmid pKFN-1718 was obtained containing the following construction $TPI_p$-LaC212spx3 signal-leader(1-47)-GluArgLeuGluLysArg-des Arg1,des-Pro2-[Ser10, Gly40, Asn41, Gly42, Glu53]-aprotinin-$TPI_T$.

The DNA sequence of the 406 bp EcoRI-XbaI fragment from pKFN-1715 and pKFN-1718 is given in Sequence Listing ID No. 19.

Plasmid pKFN-1718 was transformed in yeast strain MT663 as described above resulting in yeast strain KFN-1739.

Culturing of the transformed strain KFN-1739 in YPD-medium, analysis for des-Arg1,Pro2-[Ser10, Gly40, Asn41, Gly42, Glu53]-aprotinin in the supernatant and production of material for toxicological studies was performed as described above.

Example 11

Production of Des-Arg1,des-Pro2-[Ser42,Glu53]-aprotinin from yeast strain KFN-1742.

The 1.8 kb AhaII-XbaI fragment and the 1.4 kb AhaII-AvaII fragment both from plasmid pKFN-306 ( see example 5) were ligated to a synthetic 141 bp AvaII-XbaI fragment encoding [Ser42, Glu53]-aprotinin.

The resulting pTZ19R-derived plasmid was pKFN-1721 .

By following the procedure of Example 1, a yeast expression plasmid pKFN-1724 was obtained containing the following construction $TPI_p$-MF$\alpha$1 signal-leader(1-85)-des-Arg1,des-Pro2-[Ser42, Glu53]-aprotinin-$TPI_T$.

The DNA sequence of the 502 bp EcoRI-XbaI fragment from pKFN-1721 and pKFN-1724 is given in Sequence Listing ID No. 21.

Plasmid pKFN-1724 was transformed in yeast strain MT663 as described above resulting in yeast strain KFN-1742.

Culturing of the transformed strain KFN-1742 in YPD-medium, analysis for des-Arg1,des-Pro2-[Ser42, Glu53]-aprotinin in the supernatant and production of material for toxicological studies was performed as described above.

Example 12

Production of Des-Arg1,des-Pro2-[Glu42, Glu53]-aprotinin from yeast strain KFN-1752.

The 1.8 kb AhaII-XbaI fragment and the 1.4 kb AhaII-AvaII fragment both from plasmid pKFN-306 ( see example 5) were ligated to a synthetic 141 bp AvaII-XbaI fragment encoding [Glu42, Glu53]-aprotinin.

The resulting pTZ19R-derived plasmid was pKFN-1762 .

By following the procedure of Example 1, a yeast expression plasmid pKFN-1765 was obtained containing the following construction TPI$_p$-MFα1 signal-leader(1-85)-des-Arg1,des-Pro2-[Glu42, Glu53]-aprotinin-TPI$_T$.

The DNA sequence of the 502 bp EcoRI-XbaI fragment from pKFN-1762 and pKFN-1765 is given in Sequence Listing ID No. 23.

Plasmid pKFN-1765 was transformed in yeast strain MT663 as described above resulting in yeast strain KFN-1752.

Culturing of the transformed strain KFN-1752 in YPD-medium analysis for des-Arg1,des-Pro2-[Glu42, Glu53]-aprotinin in the supernatant and production of material for toxicological studies was performed as described above.

Example 15

Production of Des-Arg1, des-Pro2-[Glu26,Ser42, Glu53]-aprotinin from yeast strain KFN-1755.

The 1.8 kb AhaII-XbaI fragment and the 1.4 kb AhaII-AvaII fragment both from plasmid pKFN-306 ( see example 5) were ligated to a synthetic 141 bp AvaII-XbaI fragment encoding [Glu26, Ser42, Glu53]-aprotinin.

The resulting pTZ19R-derived plasmid was pKFN-1768 .

By following the procedure of Example 1, a yeast expression plasmid pKFN-1770 was obtained containing the following construction TPI$_p$-MFα1 signal-leader(1-85)-des-Arg1,des-Pro2-[Glu26, Ser42, Glu53]-aprotinin-TPI$_T$.

The DNA sequence of the 502 bp EcoRI-XbaI fragment from pKFN-1768 and pKFN-1770 is given in Sequence Listing ID No. 25.

Plasmid pKFN-1770 was transformed in yeast strain MT663 as described above resulting in yeast strain KFN-1755.

Culturing of the transformed strain KFN-1755 in YPD-medium, analysis for des-Arg1,des-Pro2-[Glu26, Ser42, Glu53]-aprotinin in the supernatant and production of material for toxicological studies was performed as described above.

Example 14

Production of Des-Arg1,des-Pro2-(Glu26, Glu42, Glu53]-aprotinin from yeast strain KFN-1756.

The 1.8 kb AhaII-XbaI fragment and the 1.4 kb AhaII-AvaII fragment both from plasmid pKFN-306 ( see example 5) were ligated to a synthetic 141 bp AvaII-XbaI fragment encoding [Glu26, Glu42, Glu53]-aprotinin.

The resulting pTZ19R-derived plasmid was pKFN-1771 .

By following the procedure of Example 1, a yeast expression plasmid pKFN-1773 was obtained containing the following construction TPI$_p$-MFα1 signal-leader(1-85) -des-Arg1,des-Pro2-[Glu26, Glu42, Glu53]-aprotinin-TPI$_T$.

The DNA sequence of the 502 bp EcoRI-XbaI fragment from pKFN-1771 and pKFN-1773 is given in Sequence Listing ID No. 27.

Plasmid pKFN-1773 was transformed in yeast strain MT663 as described above resulting in yeast strain KFN-1756.

Culturing of the transformed strain KFN-1756 in YPD-medium, analysis for des-Arg1,des-Pro2-[Ser42, Glu42, Glu53]-aprotinin in the supernatant and production of material for toxicological studies was performed as described above.

Example 15

Toxicological Screening of Aprotinin Analogues by Single-Dose Intravenous Administration to Wistar Rats

**Material**

The following aprotinin analogues with a reduced positive net charge and thermal stability compared to recombinant aprotinin (1-58) were selected for toxicological screening: KFN-1512, KFN-1514, KFN-1544, KFN-1545, KFN-1547, KFN-1660, and KFN-1661. Their main characteristics as seen from a toxicological point of view, are shown in Table 1. Data for recombinant aprotinin are shown for comparison. The denaturation temperature is shown as an indication of biological stability.

Table 1.

| General Information | | | |
|---|---|---|---|
| KFN-Type | Chain Length | Net Charge | Denaturation T, °C |
| rAprotinin | 1-58 | +6 | >100 |
| 1512 | 1-58 | -2 | 87 |
| 1514 | 1-58 | 0 | 88 |
| 1544 | 1-58 | +2 | 98 |
| 1545 | 1-58 | 0 | 93 |
| 1547 | 1-58 | +2 | 86 |
| 1660 | 3-58 | +2 | 77 |
| 1661 | 3-58 | +3 | 79 |
| 1735 | 3-58 | -1 | 68 |
| 1737 | 3-58 | 0 | 70 |
| 1739 | 3-58 | +1 | 81 |
| 1742 | 3-58 | +2 | 71 |
| 1752 | 3-58 | +1 | |
| 1755 | 3-58 | 0 | 68 |
| 1756 | 3-58 | -1 | 70 |

**Design**

On Day 1 of the screening of each analogue, groups of 2 male and 2 female rats received 33, 100, 300, or 900 mg analogue/kg body weight. Two similarly constituted control groups received physiological saline or physiological saline acidified with hydrochloric acid to an approximate pH 4.5. The latter solution served as vehicle. The dose volume was 10 ml/kg body weight in all cases. The rats were observed for 7 days and killed on Day 8. At autopsy the kidneys were weighed and prepared for histopathology. Response variables are shown in the heading of Table 2.

**Results**

Results from individual screenings are summarized in Table 2. Data for recombinant aprotinin are included for comparison (dosage: 11-300 mg/kg). KFN-1512 could not be dissolved as required for administration of the top dose (900 mg/kg).

A single animal died at 900 mg KFN-1545/kg. Apart from this no fatalities were seen.

No histopathological kidney change was seen after administration (300 mg/kg body weight) of KFN-1512, KFN-1544, KFN-1545, and KFN-1660. Furthermore, no histopathological kidney change was seen after administration of 900 mg/kg body weight of KFN-1514, KFN-1547, and KFN-1661. Thus all analogues had no-toxic-effect levels concerning histopathological kidney change of 300 mg/kg or above, as compared to 11 mg/kg for aprotinin.

With respect to the other response variables the analogues equaled or were superior to aprotinin.

Table 2    No-toxic-effect levels by response variable, mg/kg

| KFN-type | Clinical Observations | | | Morta-lity | Macro-scopic obser-vations | Micro-scopic obser-vations | Body weight Day 8 | Kidney weight Day 8 |
|---|---|---|---|---|---|---|---|---|
| | 0-30 min after dosage | 2 hours after dosage | Daily | | | | | |
| rAproti-nin [1,2] | 33 | 300 | 300 | 300 | 33 | 11 | 100 | 100 |
| 1512 [1] | 33 | 300 | 300 | 300 | 300 | 300 | 300 | 300 |
| 1514 | 900 | 900 | 900 | 900 | 900 | 900 | 900 | 900 |
| 1544 | 33 | 100 | 100 | 900 | 300 | 300 | 900 | 300 |
| 1545 | 33 | 900 | 300 | 300 | 300 | 300 | 300 | 300 |
| 1547 | 33 | 300 | 900 | 900 | 900 | 900 | 900 | 900 |
| 1660 | 300 | 900 | 900 | 900 | 900 | 900 | 900 | 900 |
| 1661 | 100 | 900 | 900 | 900 | 300 | 900 | 900 | 900 |

[1,2]    Top Dose  =  300 mg/kg
[2]     Low Dose  =   11 mg/kg

EP 0 551 329 B1

**Conclusion**

The toxicity profile of aprotinin analogues assessed by single-dose intravenous screening in Wistar rats was improved to a varying degree as compared to the toxicity profile of aprotinin. All aprotinin analogues had no-nephrotoxic-effect levels of 300 mg/kg or more. KFN-1514 had an overall no-toxic-effect level of 900 mg/kg, equaling the top dose.

Example 16

Elimination and Distribution of Recombinant Aprotinin and Aprotinin Analogues

**Materials**

Recombinant authentic aprotinin and the analogues produced according to Examples 1-7 were dissolved in 0.9% NaCl in order to obtain a dose volumen of 1 μl/g rat. The concentrations of the injection solutions were controls analyzed by methods given in the method section.

**Methods**

Female Wistar rats, weighing 200-230 g were used. Aprotinin and analogues were tested in two different models, 1) anaesthetized and 2) unanaesthetized rats.

**Anaesthetized rats**

The rats were anaesthetized by intraperitoneal injection of pentobarbital sodium. The carotid artery and the jugular vein were exposed and cannulated with polyethylene catheters (PE-50, Intramedic). The carotid catheter was connected to a perfusor (B. Braun) for infusion of 3.8 ml 0.9% NaCl/h, and to a blood pressure transducer. Changes in blood pressure were recorded by using a chart recorder (Kipp & Zonen, BD 9). The analogues were administered as bolus injections over 15 seconds through the jugular catheter.

Blood samples were obtained from the carotid catheter at 3, 10, 20, 40, and 60 minutes after administration. The samples (0.45 ml) were collected in 3 ml test tubes containing 50 μl 0.13 M sodium citrate and centrifuged. Plasma was stored at -20°C until analysis. Sixty minutes after administration the rats were killed with an excessive dose of pentobatbital sodium and the kidneys and liver were removed, weighed , and stored at - 80°C.

**Unanaesthetized rats**

An oral dose of 2 ml destilled $H_2O$ was given prior to the administration of analogues. The analogues were given intravenously as bolus injections into a tail vein by using an intravenous catheter (Venflon 22 G, Viggo-Spectramed, Helsingborg, Sweden). After administration, the catheter was flushed by 0.5 ml 0.9% NaCl and removed. A plaster was applied on the injection site in order to avoid bleeding from the tail.

The rat was then placed in a metabolism cage in order to collect the urine produced.

After 3 hours the rat was killed by administration of $CO_2/O_2$ (9/1) into the cage, and the kidneys and liver were removed and stored at -80°C until analysis. During the $CO_2$-administration, the rat emptied the urinary bladder and, after the rat was removed, the metabolism cage was rinsed with 0.9% NaCl in order to obtain a total urine-NaCl volume of 25 ml.

**Preparation of homogenates**

One kidney (approx. 1 g) and approximately 2 g liver tissue were placed in separate 10 ml plastic test tubes and 2 ml 0.9% NaCl were added. The tissues were homogenized 5 min by using a High Intensity Ultrasonic Processor (Model VC50, solics & Materials Inc. Danbury CT, USA). The kidney and liver homogenates were then diluted with saline in order to obtain a total volume of 10-25 and 4 ml, respectively.

**Methods of analysis**

The levels of aprotinin and analogues in plasma, liver homogenates and injection solutions were measured photometrically on a Cobas Fara II (Roche). Briefly, plasma, homogenates or injection solutions were precipitated with acid in order to remove other kallikrein inhibitors than aprotinin. The kallikrein inhibitory activity in the sample were measured by using kallikrein from porcine pancreas (Sigma K 3627) and the chromogenic substrate S2266 (Kabi).

The levels in kidney homogenates and urine were measured by the same method, except for the precipitation step which was omitted, since the intrinsic kallikrein inhibitory activity in the diluted homogenates and urine was negligible. Separate standard curves were employed for each analogue in each medium.

**Study Design**

14 groups of anaesthetized and 14 groups of unanaesthetized rats were studied. A dose of 1.56 μmoles (approximately 10 mg) aprotinin or aprotinin analogue per kg body weight were administered to each rat. Basal data on the 28 groups are given in table III.

Table III

| Groups | n | BW | KW | LW |
|---|---|---|---|---|
| rAprotinin A | 5 | 251.8 | 0.98 | 9.9 |
| KFN 1512 A | 4 | 230.0 | 0.84 | 9.6 |
| KFN 1514 A | 4 | 220.5 | 0.85 | 8.6 |
| KFN 1544 A | 4 | 226.0 | 0.97 | 9.3 |
| KFN 1545 A | 4 | 224.8 | 0.92 | 9.2 |
| KFN 1547 A | 4 | 229.0 | 0.75 | 8.6 |
| KFN 1660 A | 4 | 220.5 | 0.93 | 9.7 |
| KFN 1661 A | 4 | 240.8 | 0.97 | 9.0 |
| rAprotinin U | 4 | 192.5 | 0.77 | 9.8 |
| KFN 1512 U | 5 | 191.0 | 0.65 | 7.2 |
| KFN 1514 U | 6 | 188.3 | 0.69 | 7.3 |
| KFN 1544 U | 5 | 190.0 | 0.64 | 6.5 |
| KFN 1545 U | 6 | 185.8 | 0.66 | 7.3 |
| KFN 1547 U | 5 | 188.0 | 0.67 | 7.3 |
| KFN 1660 U | 6 | 205.0 | 0.77 | 8.5 |
| KFN 1661 U | 6 | 204.2 | 0.80 | 8.1 |
| BW: Body weight (g). KW: Kidney weight (g) | | | | |
| LW: Liver weight (g). A: anaesthetized rat model | | | | |
| U: Unanaesthetized rat model. | | | | |

**Statistical evaluation**

Spearman's rank sum correlation test was used for the statistical evaluation.

Table 4    Aprotinin Analogues:
           Content of Inhibitory Activity in Kidneys and Urine after i.v. Administration
           to Rats

| Analogue ID | Net Charge | Denaturation[2] Temperature °C (max) | Content in Urine (3 h) % of dose | Content in Kidneys (1 h) % of dose | Content in Kidneys (3 h) % of dose | Accumulation Index[1] |
|---|---|---|---|---|---|---|
| Aprotinin | +6 | >100 | 2 | 21 | 44 | 2.10 |
| KFN 1512 | −2 | 87 | 51 | 2 | 2 | 1 |
| KFN 1514 | 0 | 88 | 36 | 8 | 11 | 1.38 |
| KFN 1544 | +2 | 98 | 42 | 17 | 23 | 1.35 |
| KFN 1545 | 0 | 93 | 41 | 14 | 28 | 2 |
| KFN 1547 | +2 | 86 | 45 | 4 | 5 | 1.25 |
| KFN 1660 | +2 | 77 | 23 | 6 | 3 | 0.5 |
| KFN 1661 | +3 | 79 | 18 | 4 | 3 | 0.75 |

[1]  The kidney accumulation index is calculated as the content of inhibitory activity after 3 hours divided by the content after 1 hour

[2]  Measurement by differential scanning calorimetry in 20 mM 2-(N-Morpholino)ethane sulfonic acid, pH 5.5

EP 0 551 329 B1

Results

**Analogues in Kidneys and Urine**

The total content in kidneys (in percent of dosage) after 1 and 3 hours and in urine after 3 h is shown in Fig. 3 and Table IV.

It appears that great differences between the analogues were found.

As regards aprotinin, the content in kidneys 1 hour after administration was approximately 20% of the dose, whereas the content increased to more than 40% after 3 hours. The excretion of aprotinin in urine was neglible.

In order to evaluate whether the excretion in urine after 3 hours was associated with the net charge of the analogues, the degree of correlation between these figures was calculated.

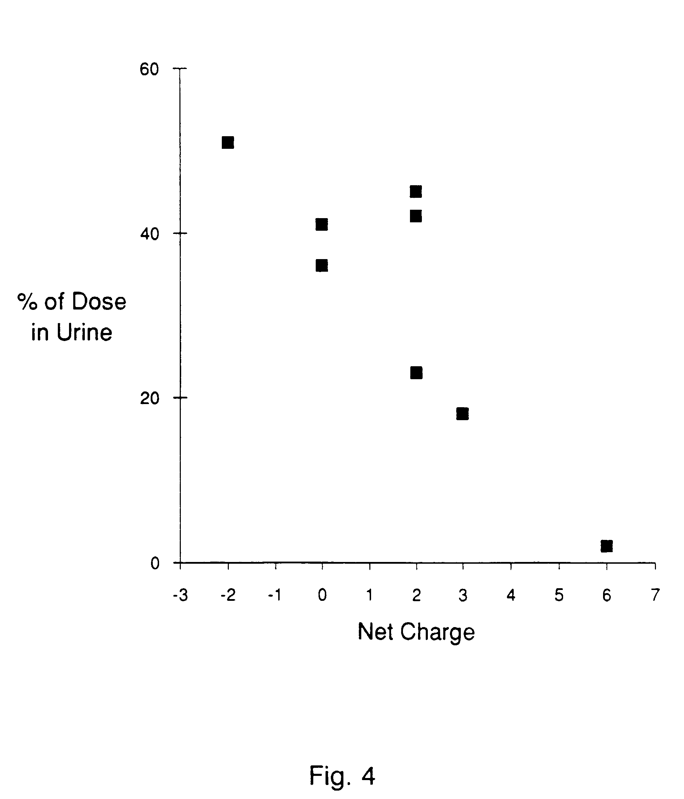

The content in urine was found to be strongly correlated with the net charges of the analogues (cf. Fig. 4).

Table V

| Analogues | Acc. index | Stab. index kidney | Denat. tp. (°C) |
|---|---|---|---|
| rAprotinin A | 2.10 | 0.90 | 100 |
| KFN 1512 A | 0.66 | 0.67 | 87 |
| KFN 1514 A | 1.32 | 0.87 | 88 |
| KFN 1544 A | 1.32 | 1.05 | 98 |
| KFN 1545 A | 1.99 | 0.71 | 93 |
| KFN 1547 A | 1.22 | 0.65 | 86 |
| KFN 1660 A | 0.50 | 0.55 | 77 |
| KFN 1661 A | 0.77 | 0.55 | 79 |

**Stability of analogues**

In order to study the stability of the analogues in kidney tissue, one kidney from 14 anaesthetized rats (one from each group) was divided into two pieces of identical weight. One piece was stored at 37°C and the other piece at 4°C. After 4 hours, the tissues were homogenized and the content of analogues was measured. A stability index was defined as the content in the piece stored at 37°C divided by the content in the piece stored at 4°C. The stability indices are given in table VI showing that rAprotinin, KFN 1514, KFN 1544, seem to be the most stable compounds as compared to e.g. KFN 1660 which appears to be more unstable.

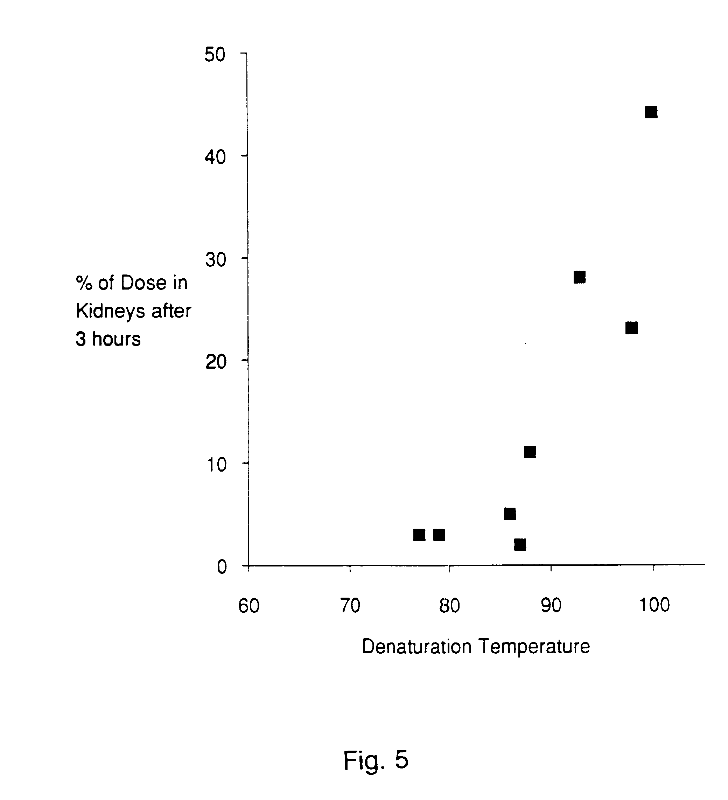

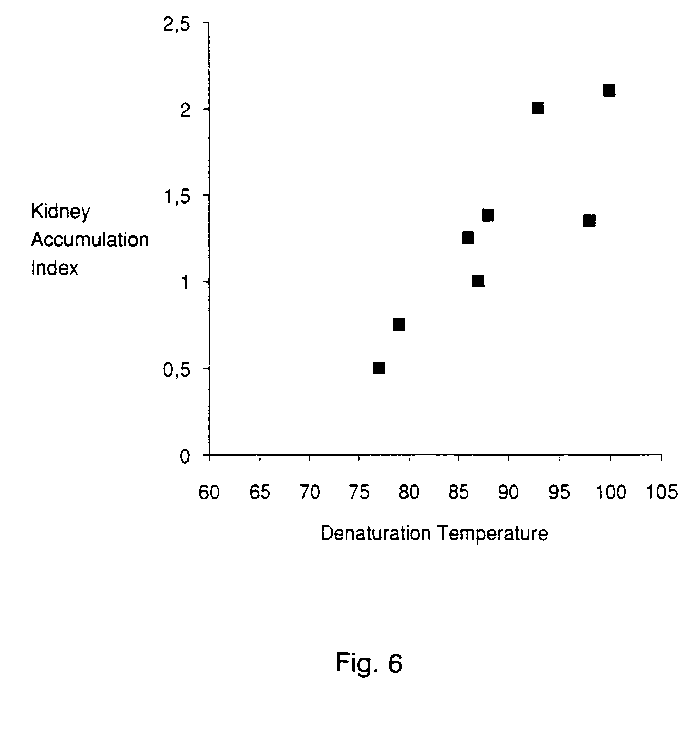

The stability of analogues has also been studied by the determination of their denaturation temperature. The denaturation temperatures were found to be highly correlated with the content in kidney tissue 3 hours after administration (Fig. 5) and with the accumulation indices (Fig. 6), but not with urine excretion.

These data suggest that the net charge may be of importance for the excretion in urine, but of minor importance for the concentration and accumulation in kidneys. On the other hand, the renal accumulation seems to be related to the denaturation temperature and to the stability of analogues in kidney tissue.

It is, however, likely that the concentrations in kidney tissue, when measured 1 hour after administration, were changed due to degradation or redistribution. Thus, it is possible that the concentrations measured e.g. 10 min after administration would correlate with the net charge.

**Conclusions**

The following conclusions were made:

1) All analogues tested were taken up by the kidneys but to varying degrees. The accumulation in the kidneys seemed to be related to the thermostability and the stability in kidney tissue, but not to the net charge of the molecules.

2) The excretion in urine seemed to be associated with the net charge of the analogues, but not with the stability.

## Claims

1. An aprotinin analogue wherein, to provide a reduced positive net charge, at least one positively charged amino acid residue outside the protease-binding site is removed or replaced with a neutral or negatively charged amino acid residue, and/or wherein at least one negatively charged amino acid residue is inserted or added, and/or wherein at least one neutral amino acid residue is replaced with a negatively charged amino acid residue, and/or wherein, to provide reduced thermal stability of the analogue in aqueous solutions at a pH of about 4-10, one or more amino acid residues outside the protease-binding site, that participate in stabilizing the analogue, are deleted or replaced with one or more other amino acid residues, and/or one or more amino acid residues, that destabilize the analogue, are added outside the protease-binding site,

   **with the exception of** such aprotinin analogues wherein the amino acid in position 1 or the amino acids in positions 1 and 2 have been deleted; Ile in position 19 is substituted by another naturally occurring amino acid residue; Lys in position 41 is substituted by another naturally occurring amino acid residue; or Arg in position 42 is substituted by Lys or Ser.

2. An aprotinin analogue according to claim 1, which has the general formula I

$$R^1 \text{ Asp Phe Cys Leu Glu Pro Pro } R^2 \text{ Thr Gly Pro Cys Lys Ala Arg}$$
$$\text{Ile Ile } R^3 \text{ Tyr Phe Tyr } R^4 \text{ Ala } R^5 \text{ Ala Gly Leu Cys } R^6 \text{ Thr Phe Val}$$
$$\text{Tyr Gly Gly Cys Arg } R^7 \ R^8 \ R^9 \text{ Asn } R^{10} \text{ Phe } R^{11} \text{ Ser Ala Glu Asp Cys}$$
$$\text{Met } R^{12} \text{ Thr Cys Gly Gly Ala}$$

$$(I)$$

   wherein

   $R^1$ is a dipeptide selected from the group consisting of Arg-Pro, Glu-Pro, Asp-Pro, Ala-Pro, Ile-Pro, Thr-Pro, His-Pro, Leu-Pro, Gly-Pro and Ser-Pro, Pro, or $R^1$ is hydrogen,

   $R^2$ is an amino acid residue selected from the group consisting of Tyr, Glu, Asp, Ser, Thr, Ala and Val,

   $R^3$ is an amino acid residue selected from the group consisting of Arg, Glu, Asp, Leu, Ser, Ala, Gln and Thr,

   $R^4$ is an amino acid residue selected from the group consisting of Asn, Glu and Asp,

   $R^5$ is an amino acid residue selected from the group consisting of Lys, Glu, Asp, Thr, Val, Ala, Ser, Phe, Gln and Gly,

   $R^6$ is an amino acid residue selected from the group consisting of Gln, Glu, Asp, Val and Ala,

   $R^7$ is an amino acid residue selected from the group consisting of Ala, Asp, Glu and Gly,

   $R^8$ is an amino acid residue selected from the group consisting of Lys, Glu, Asp, Asn, Ser, Thr and Ala,

   $R^9$ is an amino acid residue selected from the group consisting of Arg, Glu, Asp, Ser, Asn, Leu, Gly, Gln, Met and Thr,

$R^{10}$ is an amino acid residue selected from the group consisting of Asn, Glu and Asp,

$R^{11}$ is an amino acid residue selected from the group consisting of Lys, Glu, Asp, Leu, Tyr, Ala, Val, Thr, Ser, Pro, His and Ile, and

$R^{12}$ is an amino acid residue selected from the group consisting of Arg, Glu, Asp, Gln, Ala, Asn, His, Gly, Ser and Thr,

with the proviso that at least one of the amino acid residues $R^1$-$R^{12}$ is different from the corresponding amino acid residue of native aprotinin and that when $R^1$ is hydrogen, at least one of the amino acid residues $R^2$ - $R^{12}$ is different from the corresponding amino acid residue of native aprotinin, except that when $R^1$ is hydrogen $R^9$ is not Ser.

3. An aprotinin analogue according to claim 1, wherein a peptide consisting essentially of one or more negatively charged or neutral amino acid residues are added at the N-terminal end of the aprotinin molecule.

4. An aprotinin analogue according to claim 1, wherein a peptide consisting essentially of one or more negatively charged or neutral amino acid residues are added at the C-terminal end of the aprotinin molecule.

5. An aprotinin analogue according to any of the preceding claims which, in addition to being modified outside the protease-binding site, is modified by substitution of one or more amino acid residues in the protease-binding site, with one or more other other amino acid residues, respectively,

**with the exception of** such aprotinin analogues wherein the amino acid in position 1 or the amino acids in positions 1 and 2 have been deleted; Ile in position 19 is substituted by another naturally occurring amino acid residue; Lys in position 41 is substituted by another naturally occurring amino acid residue; or Arg in position 42 is substituted by Lys or Ser; and

wherein Gly in position 12 is substituted by another naturally occurring amino acid residue; Pro in position 13 is substituted by another naturally occurring amino acid residue; Cys in position 14 is substituted by another naturally occurring amino acid residue; Lys in position 15 is substituted by Arg, Val, Thr, Ile, Leu, Phe, Gly, Ser, Met, Trp, Tyr or Ala; Ala in position 16 is Gly; Arg in position 17 is substituted by another naturally occurring amino acid residue; Ile in position 18 is substituted by Leu, Met, Val or Phe; or Cys in position 38 is substituted by another naturally occurring amino acid residue.

6. An aprotinin analogue according to claim 5, which has the general formula II

```
R¹ Asp Phe Cys Leu Glu Pro Pro R² Thr Gly Pro Cys R¹³ R¹⁴ R¹⁵ R¹⁶
R¹⁷ R³ Tyr Phe Tyr R⁴ Ala R⁵ Ala Gly Leu Cys R⁶ Thr Phe R¹⁸ Tyr R¹⁹
Gly Cys R²⁰ R⁷ R⁸ R⁹ Asn R¹⁰ Phe R¹¹ Ser Ala Glu Asp Cys Met R¹² Thr
Cys Gly Gly Ala
```

$$(II)$$

wherein $R^1$-$R^{12}$ are as defined in claim 2,

$R^{13}$ is an amino acid residue selected from the group consisting of Lys, Arg, Glu, Leu, Met, Tyr and Phe,

$R^{14}$ is an amino acid residue selected from the group consisting of Ala and Gly,

$R^{15}$ is an amino acid residue selected from the group consisting of Arg, Ala, Gly, Lys, Leu, Met, Phe, Tyr, Ile and Asn,

$R^{16}$ is an amino acid residue selected from the group consisting of Ile, Met, Leu, Phe, Thr and Glu,

$R^{17}$ is an amino acid residue selected from the group consisting of Ile, Leu, Lys, Gln, Glu, Ser, Arg, Thr and Asn,

$R^{18}$ is an amino acid residue selected from the group consisting of Val, Thr, Leu, Ser, Tyr, Gln, His, Pro, Phe, Asn, Ile and Lys,

$R^{19}$ is an amino acid residue selected from the group consisting of Gly, Thr, and Ser, and

$R^{20}$ is an amino acid residue selected from the group consisting of Gln, Lys, Met, Asn, Leu, Gly and Glu,

with the proviso that at least one of the amino acid residues $R^1$-$R^{12}$ and at least one of the amino acid residues $R^{13}$-$R^{20}$ are different from the corresponding amino acid residue of native aprotinin and that when $R^1$ is hydrogen, $R^{15}$ is not Ala, $R^{17}$ is not Glu, and $R^9$ is not Ser, and that when $R^{15}$ is Ala, $R^9$ is not Ser.

7. An aprotinin analogue according to claim 2, wherein $R^1$ is Glu-Pro, $R^5$ is Glu, $R^8$ is Glu, $R^{11}$ is Glu, and $R^2$, $R^3$, R4, $R^6$, $R^7$, $R^9$, $R^{10}$ and $R^{12}$ are as in the native aprotinin sequence.

8. An aprotinin analogue according to claim 2, wherein $R^1$ is Glu-Pro, $R^9$ is Glu, $R^{11}$ is Glu, and $R^2$, $R^3$, $R^4$, $R^6$, $R^7$, $R^8$, $R^{10}$ and $R^{12}$ are as in the native aprotinin sequence.

9. An aprotinin analogue according to claim 2, wherein $R^9$ is Glu, $R^{11}$ is Glu, and $R^1$, $R^2$, $R^3$, $R^4$, $R^5$ $R^6$, $R^7$, $R^8$, $R^{10}$ and $R^{12}$ are as in the native aprotinin sequence.

10. An aprotinin analogue according to claim 2, wherein $R^2$ is Ser, R4 is Asp, $R^5$ is Thr, $R^6$ is Glu, $R^8$ is Asn, $R^{12}$ is Glu, and $R^1$, $R^3$, $R^7$, $R^9$, $R^{10}$, and $R^{11}$ are as in the native aprotinin sequence.

11. An aprotinin analogue according to claim 2, wherein $R^2$ is Ser, $R^3$ is Leu, $R^7$ is Gly, $R^8$ is Asn, $R^9$ is Gly, $R^{10}$ is Gln, $R^{11}$ is Tyr, and $R^1$, $R^4$, $R^5$ $R^6$, and $R^{12}$ are as in the native aprotinin sequence.

12. An aprotinin analogue according to claim 2, wherein $R^1$ is hydrogen, $R^9$ is Ser, $R^{11}$ is Glu, and $R^2$, $R^3$, $R^4$, $R^5$, $R^6$, $R^7$, $R^8$, $R^{10}$ and $R^{12}$ are as in the native aprotinin sequence.

13. An aprotinin analogue according to claim 2, wherein $R^1$ is hydrogen, $R^9$ is Ser, $R^{11}$ is Ala, and $R^2$, $R^3$, $R^4$, $R^5$, $R^6$, $R^7$, $R^8$, $R^{10}$ and $R^{12}$ are as in the native aprotinin sequence.

14. An aprotinin analogue according to claim 2, wherein $R^1$ is hydrogen, $R^2$ is Ser, $R^4$ is Asp, $R^5$ is Thr, $R^6$ is Glu, $R^8$ is Asn, $R^{12}$ is Glu, and $R^3$, $R^7$, $R^9$, $R^{10}$ and $R^{11}$ are as in the native aprotinin sequence.

15. An aprotinin analogue according to claim 2, wherein $R^1$ is hydrogen, $R^4$ is Asp, $R^5$ is Thr, $R^6$ is Glu, $R^{12}$ is Glu, and $R^2$, $R^3$, $R^7$, $R^8$, $R^9$, $R^{10}$ and $R^{11}$ are as in the native aprotinin sequence.

16. An aprotinin analogue according to claim 2, wherein $R^1$ is hydrogen, $R^2$ is Ser, $R^7$ is Gly, $R^8$ is Asn, $R^9$ is Gly, $R^{12}$ is Glu, and $R^3$, $R^4$, $R^5$ $R^6$, $R^{10}$ and $R^{11}$ are as in the native aprotinin sequence.

17. An aprotinin analogue according to claim 2, wherein $R^1$ is hydrogen, $R^9$ is Ser, $R^{12}$ is Glu, and $R^2$, $R^3$, R4, $R^5$, $R^6$, $R^7$, $R^8$, $R^{10}$ and $R^{11}$ are as in the native aprotinin sequence.

18. An aprotinin analogue according to claim 2, wherein $R^1$ is hydrogen, $R^9$ is Glu, $R^{12}$ is Glu, and $R^2$, $R^3$, $R^4$, $R^5$, $R^6$, $R^7$, $R^8$, $R^{10}$ and $R^{11}$ are as in the native aprotinin sequence.

19. An aprotinin analogue according to claim 2, wherein $R^1$ is hydrogen, $R^5$ is Glu, $R^9$ is Ser, $R^{12}$ is Glu, and $R^2$, $R^3$, $R^4$, $R^6$, $R^7$, $R^8$, $R^{10}$ and $R^{11}$ are as in the native aprotinin sequence.

20. An aprotinin analogue according to claim 2, wherein $R^1$ is hydrogen, $R^5$ is Glu, $R^9$ is Glu, $R^{12}$ is Glu, and $R^2$, $R^3$, $R^4$, $R^6$, $R^7$, $R^8$, $R^{10}$ and $R^{11}$ are as in the native aprotinin sequence.

21. A DNA construct comprising a DNA sequence encoding an aprotinin analogue according to any of claims 1-20.

22. A recombinant expression vector comprising a DNA construct according to claim 21.

23. A cell containing an expression vector according to claim 22.

24. A method of producing an aprotinin analogue according to any of claims 1-20, wherein a cell according to claim 23 is cultured under conditions conducive to the expression of the aprotinin analogue, and the resulting analogue is recovered from the culture.

25. A pharmaceutical composition comprising an aprotinin analogue according to any of claims 1-20 together with a pharmaceutically acceptable carrier or excipient.

26. Use of an aprotinin analogue according to any of claims 1-20 for the manufacture of a medicament with reduced nephrotoxicity compared to native aprotinin.

27. Use of an aprotinin analogue according to any of claims 1-20 for the manufacture of a medicament which, on administration, gives rise to a reduced incidence of anaphylactoid reactions compared to those experienced with native aprotinin.

**Patentansprüche**

1. Aprotinin-Analogon, wobei, um eine reduzierte positive Nettoladung zu erhalten, wenigstens ein positiv geladener Aminosäurerest außerhalb der Protease-Bindungsstelle entfernt oder durch einen neutralen oder negativ geladenen Aminosäurerest ersetzt ist und/oder wobei wenigstens ein negativ geladener Aminosäurerest insertiert oder hinzugefügt ist und/oder wobei wenigstens ein neutraler Aminosäurerest durch einen negativ geladenen Aminosäurerest ersetzt ist und/oder wobei, um eine reduzierte thermische Stabilität des Analogons in wäßrigen Lösungen bei einem pH-Wert von etwa 4-10 zu erhalten, ein oder mehrere Aminosäurereste außerhalb der Protease-Bindungsstelle, die an der Stabilisierung des Analogons beteiligt sind, deletiert oder durch einen oder mehrere andere Aminosäurereste ersetzt sind und/oder ein oder mehrere Aminosäurereste, die das Analogon destabilisieren, außerhalb der Protease-Bindungsstelle hinzugefügt sind,

   mit Ausnahme solcher Aprotinin-Analoga, bei denen die Aminosäure in Position 1 oder die Aminosäuren in den Positionen 1 und 2 deletiert sind, Ile in Position 19 durch einen anderen natürlich vorkommenden Aminosäurerest ersetzt ist, Lys in Position 41 durch einen anderen natürlich vorkommenden Aminosäurerest ersetzt ist oder Arg in Position 42 durch Lys oder Ser ersetzt ist.

2. Aprotinin-Analogon gemäß Anspruch 1, das die allgemeine Formel I

$R^1$ Asp Phe Cys Leu Glu Pro Pro $R^2$ Thr Gly Pro Cys Lys Ala Arg Ile Ile $R^3$ Tyr Phe Tyr $R^4$ Ala $R^5$ Ala Gly Leu Cys $R^6$ Thr Phe Val Tyr Gly Gly Cys Arg $R^7$ $R^8$ $R^9$ Asn $R^{10}$ Phe $R^{11}$ Ser Ala Glu Asp Cys Met $R^{12}$ Thr Cys Gly Gly Ala

$$(I)$$

aufweist, wobei

$R^1$ ein Dipeptid, das aus der Gruppe ausgewählt ist, die aus Arg-Pro, Glu-Pro, Asp-Pro, Ala-Pro, Ile-Pro, Thr-Pro, His-Pro, Leu-Pro, Gly-Pro und Ser-Pro besteht, Pro oder Wasserstoff ist,

$R^2$ ein Aminosäurerest ist, der aus der Gruppe ausgewählt ist, die aus Tyr, Glu, Asp, Ser, Thr, Ala und Val besteht,

$R^3$ ein Aminosäurerest ist, der aus der Gruppe ausgewählt ist, die aus Arg, Glu, Asp, Leu, Ser, Ala, Gln und Thr besteht,

$R^4$ ein Aminosäurerest ist, der aus der Gruppe ausgewählt ist, die aus Asn, Glu und Asp besteht,

$R^5$ ein Aminosäurerest ist, der aus der Gruppe ausgewählt ist, die aus Lys, Glu, Asp, Thr, Val, Ala, Ser, Phe, Gln und Gly besteht,

$R^6$ ein Aminosäurerest ist, der aus der Gruppe ausgewählt ist, die aus Gln, Glu, Asp, Val und Ala besteht,

$R^7$ ein Aminosäurerest ist, der aus der Gruppe ausgewählt ist, die aus Ala, Asp, Glu und Gly besteht,

$R^8$ ein Aminosäurerest ist, der aus der Gruppe ausgewählt ist, die aus Lys, Glu, Asp, Asn, Ser, Thr und Ala besteht,

$R^9$ ein Aminosäurerest ist, der aus der Gruppe ausgewählt ist, die aus Arg, Glu, Asp, Ser, Asn, Leu, Gly, Gln, Met und Thr besteht,

R$^{10}$ ein Aminosäurerest ist, der aus der Gruppe ausgewählt ist, die aus Asn, Glu und Asp besteht,

R$^{11}$ ein Aminosäurerest ist, der aus der Gruppe ausgewählt ist, die aus Lys, Glu, Asp, Leu, Tyr, Ala, Val, Thr, Ser, Pro, His und Ile besteht, und

R$^{12}$ ein Aminosäurerest ist, der aus der Gruppe ausgewählt ist, die aus Arg, Glu, Asp, Gln, Ala, Asn, His, Gly, Ser und Thr besteht,

mit der Maßgabe, daß wenigstens einer der Aminosäurereste R$^1$-R$^{12}$ von dem entsprechenden Aminosäurerest des nativen Aprotinins verschieden ist und daß, wenn R$^1$ Wasserstoff ist, wenigstens einer der Aminosäurereste R$^2$-R$^{12}$ von dem entsprechenden Aminosäurerest des nativen Aprotinins verschieden ist, außer daß R$^9$ nicht Ser ist, wenn R$^1$ Wasserstoff ist.

3. Aprotinin-Analogon gemäß Anspruch 1, wobei ein Peptid, das im wesentlichen aus einem oder mehreren negativ geladenen oder neutralen Aminosäureresten besteht, am N-Terminus des Aprotininmoleküls hinzugefügt ist.

4. Aprotinin-Analogon gemäß Anspruch 1, wobei ein Peptid, das im wesentlichen aus einem oder mehreren negativ geladenen oder neutralen Aminosäureresten besteht, am C-Terminus des Aprotininmoleküls hinzugefügt ist.

5. Aprotinin-Analogon gemäß einem der vorstehenden Ansprüche, das neben der Modifikation außerhalb der Protease-Bindungsstelle noch durch Ersatz von einem oder mehreren Aminosäureresten in der Protease-Bindungsstelle durch einen bzw. mehrere andere Aminosäurereste modifiziert ist,
mit Ausnahme solcher Aprotinin-Analoga, bei denen die Aminosäure in Position 1 oder die Aminosäuren in den Positionen 1 und 2 deletiert sind, Ile in Position 19 durch einen anderen natürlich vorkommenden Aminosäurerest ersetzt ist, Lys in Position 41 durch einen anderen natürlich vorkommenden Aminosäurerest ersetzt ist oder Arg in Position 42 durch Lys oder Ser ersetzt ist, und
wobei Gly in Position 12 durch einen anderen natürlich vorkommenden Aminosäurerest ersetzt ist, Pro in Position 13 durch einen anderen natürlich vorkommenden Aminosäurerest ersetzt ist, Cys in Position 14 durch einen anderen natürlich vorkommenden Aminosäurerest ersetzt ist, Lys in Position 15 durch Arg, Val, Thr, Ile, Leu, Phe, Gly, Ser, Met, Trp, Tyr oder Ala ersetzt ist, Ala in Position 16 Gly ist, Arg in Position 17 durch einen anderen natürlich vorkommenden Aminosäurerest ersetzt ist, Ile in Position 18 durch Leu, Met, Val oder Phe ersetzt ist oder Cys in Position 38 durch einen anderen natürlich vorkommenden Aminosäurerest ersetzt ist.

6. Aprotinin-Analogon gemäß Anspruch 5, das die allgemeine Formel II

R$^1$ Asp Phe Cys Leu Glu Pro Pro R$^2$ Thr Gly Pro Cys R$^{13}$ R$^{14}$ R$^{15}$ R$^{16}$ R$^{17}$ R$^3$ Tyr Phe Tyr R$^4$ Ala R$^5$ Ala Gly Leu Cys R$^6$ Thr Phe R$^{18}$ Tyr R$^{19}$ Gly Cys R$^{20}$ R$^7$ R$^8$ R$^9$ Asn R$^{10}$ Phe R$^{11}$ Ser Ala Glu Asp Cys Met R$^{12}$ Thr Cys Gly Gly Ala

(II)

aufweist, wobei R$^1$-R$^{12}$ wie in Anspruch 2 definiert sind,

R$^{13}$ ein Aminosäurerest ist, der aus der Gruppe ausgewählt ist, die aus Lys, Arg, Glu, Leu, Met, Tyr und Phe besteht,

R$^{14}$ ein Aminosäurerest ist, der aus der Gruppe ausgewählt ist, die aus Ala und Gly besteht,

R$^{15}$ ein Aminosäurerest ist, der aus der Gruppe ausgewählt ist, die aus Arg, Ala, Gly, Lys, Leu, Met, Phe, Tyr, Ile und Asn besteht,

R$^{16}$ ein Aminosäurerest ist, der aus der Gruppe ausgewählt ist, die aus Ile, Met, Leu, Phe, Thr und Glu besteht,

R$^{17}$ ein Aminosäurerest ist, der aus der Gruppe ausgewählt ist, die aus Ile, Leu, Lys, Gln, Glu, Ser, Arg, Thr

und Asn besteht,

$R^{18}$ ein Aminosäurerest ist, der aus der Gruppe ausgewählt ist, die aus Val, Thr, Leu, Ser, Tyr, Gln, His, Pro, Phe, Asn, Ile und Lys besteht,

$R^{19}$ ein Aminosäurerest ist, der aus der Gruppe ausgewählt ist, die aus Gly, Thr und Ser besteht, und

$R^{20}$ ein Aminosäurerest ist, der aus der Gruppe ausgewählt ist, die aus Gln, Lys, Met, Asn, Leu, Gly und Glu besteht,

mit der Maßgabe, daß wenigstens einer der Aminosäurereste $R^1$-$R^{12}$ und wenigstens einer der Aminosäurereste $R^{13}$-$R^{20}$ von dem entsprechenden Aminosäurerest des nativen Aprotinins verschieden sind und daß, wenn $R^1$ Wasserstoff ist, $R^{15}$ nicht Ala ist, $R^{17}$ nicht Glu ist und $R^9$ nicht Ser ist und daß, wenn $R^{15}$ Ala ist, $R^9$ nicht Ser ist.

7.  Aprotinin-Analogon gemäß Anspruch 2, wobei $R^1$ Glu-Pro ist, $R^5$ Glu ist, $R^8$ Glu ist, $R^{11}$ Glu ist und $R^2$, $R^3$, $R^4$, $R^6$, $R^7$, $R^9$, $R^{10}$ und $R^{12}$ wie in der nativen Aprotininsequenz sind.

8.  Aprotinin-Analogon gemäß Anspruch 2, wobei $R^1$ Glu-Pro ist, $R^9$ Glu ist, $R^{11}$ Glu ist und $R^2$, $R^3$, $R^4$, $R^6$, $R^7$, $R^8$, $R^{10}$ und $R^{12}$ wie in der nativen Aprotininsequenz sind.

9.  Aprotinin-Analogon gemäß Anspruch 2, wobei $R^9$ Glu ist, $R^{11}$ Glu ist und $R^1$, $R^2$, $R^3$, $R^4$, $R^5$, $R^6$, $R^7$, $R^8$, $R^{10}$ und $R^{12}$ wie in der nativen Aprotininsequenz sind.

10. Aprotinin-Analogon gemäß Anspruch 2, wobei $R^2$ Ser ist, $R^4$ Asp ist, $R^5$ Thr ist, $R^6$ Glu ist, $R^8$ Asn ist, $R^{12}$ Glu ist und $R^1$, $R^3$, $R^7$, $R^9$, $R^{10}$ und $R^{11}$ wie in der nativen Aprotininsequenz sind.

11. Aprotinin-Analogon gemäß Anspruch 2, wobei $R^2$ Ser ist, $R^3$ Leu ist, $R^7$ Gly ist, $R^8$ Asn ist, $R^9$ Gly ist, $R^{10}$ Glu ist, $R^{11}$ Tyr ist und $R^1$, $R^4$, $R^5$, $R^6$ und $R^{12}$ wie in der nativen Aprotininsequenz sind.

12. Aprotinin-Analogon gemäß Anspruch 2, wobei $R^1$ Wasserstoff ist, $R^9$ Ser ist, $R^{11}$ Glu ist und $R^2$, $R^3$, $R^4$, $R^5$, $R^6$, $R^7$, $R^8$, $R^{10}$ und $R^{12}$ wie in der nativen Aprotininsequenz sind.

13. Aprotinin-Analogon gemäß Anspruch 2, wobei $R^1$ Wasserstoff ist, $R^9$ Ser ist, $R^{11}$ Ala ist und $R^2$, $R^3$, $R^4$, $R^5$, $R^6$, $R^7$, $R^8$, $R^{10}$ und $R^{12}$ wie in der nativen Aprotininsequenz sind.

14. Aprotinin-Analogon gemäß Anspruch 2, wobei $R^1$ Wasserstoff ist, $R^2$ Ser ist, $R^4$ Asp ist, $R^5$ Thr ist, $R^6$ Glu ist, $R^8$ Asn ist, $R^{12}$ Glu ist und $R^3$, $R^7$, $R^9$, $R^{10}$ und $R^{11}$ wie in der nativen Aprotininsequenz sind.

15. Aprotinin-Analogon gemäß Anspruch 2, wobei $R^1$ Wasserstoff ist, $R^4$ Asp ist, $R^5$ Thr ist, $R^6$ Glu ist, $R^{12}$ Glu ist und $R^2$, $R^3$, $R^7$, $R^8$, $R^9$, $R^{10}$ und $R^{11}$ wie in der nativen Aprotininsequenz sind.

16. Aprotinin-Analogon gemäß Anspruch 2, wobei $R^1$ Wasserstoff ist, $R^2$ Ser ist, $R^7$ Gly ist, $R^8$ Asn ist, $R^9$ Gly ist, $R^{12}$ Glu ist und $R^3$, $R^4$, $R^5$ $R^6$, $R^{10}$ und $R^{11}$ wie in der nativen Aprotininsequenz sind.

17. Aprotinin-Analogon gemäß Anspruch 2, wobei $R^1$ Wasserstoff ist, $R^9$ Ser ist, $R^{12}$ Glu ist und $R^2$, $R^3$, $R^4$, $R^5$, $R^6$, $R^7$, $R^8$, $R^{10}$ und $R^{11}$ wie in der nativen Aprotininsequenz sind.

18. Aprotinin-Analogon gemäß Anspruch 2, wobei $R^1$ Wasserstoff ist, $R^9$ Glu ist, $R^{12}$ Glu ist und $R^2$, $R^3$, $R^4$, $R^5$, $R^6$, $R^7$, $R^8$, $R^{10}$ und $R^{11}$ wie in der nativen Aprotininsequenz sind.

19. Aprotinin-Analogon gemäß Anspruch 2, wobei $R^1$ Wasserstoff ist, $R^5$ Glu ist, $R^9$ Ser ist, $R^{12}$ Glu ist und $R^2$, $R^3$, $R^4$, $R^6$, $R^7$, $R^8$, $R^{10}$ und $R^{11}$ wie in der nativen Aprotininsequenz sind.

20. Aprotinin-Analogon gemäß Anspruch 2, wobei $R^1$ Wasserstoff ist, $R^5$ Glu ist, $R^9$ Glu ist, $R^{12}$ Glu ist und $R^2$, $R^3$, $R^4$, $R^6$, $R^7$, $R^8$, $R^{10}$ und $R^{11}$ wie in der nativen Aprotininsequenz sind.

21. DNA-Konstrukt, das eine DNA-Sequenz umfaßt, die ein Aprotinin-Analogon gemäß einem der Ansprüche 1 bis 20 codiert.

**22.** Rekombinanter Expressionsvektor, der ein DNA-Konstrukt gemäß Anspruch 21 umfaßt.

**23.** Zelle, die einen Expressionsvektor gemäß Anspruch 22 enthält.

**24.** Verfahren zur Herstellung eines Aprotinin-Analogons gemäß einem der Ansprüche 1 bis 20, wobei eine Zelle gemäß Anspruch 23 unter Bedingungen kultiviert wird, die der Expression des Aprotinin-Analogons förderlich sind, und das resultierende Analogon aus der Kultur gewonnen wird.

**25.** Pharmazeutische Zusammensetzung, die ein Aprotinin-Analogon gemäß einem der Ansprüche 1 bis 20 zusammen mit einem pharmazeutisch annehmbaren Träger oder Verdünnungsmittel umfaßt.

**26.** Verwendung eines Aprotinin-Analogons gemäß einem der Ansprüche 1 bis 20 zur Herstellung eines Medikaments mit im Vergleich zu nativem Aprotinin reduzierter Nephrotoxizität.

**27.** Verwendung eines Aprotinin-Analogons gemäß einem der Ansprüche 1 bis 20 zur Herstellung eines Medikaments, das beim Verabreichen zu einem reduzierten Auftreten anaphylactoider Reaktionen im Vergleich zu denen, die man mit nativem Aprotinin erhält, führt.

## Revendications

**1.** Analogue d'aprotinine dans lequel, pour obtenir une charge nette positive réduite, au moins un résidu d'acide aminé positivement chargé en dehors du site de liaison à la protéase est retiré ou remplacé par un résidu d'acide aminé neutre ou négativement chargé, et/ou dans lequel au moins un résidu d'acide aminé négativement chargé est inséré ou ajouté, et/ou dans lequel au moins un résidu d'acide aminé neutre est remplacé par un résidu d'acide aminé négativement chargé, et/ou dans lequel, pour obtenir une stabilité thermique réduite de l'analogue dans des solutions aqueuses à un pH d'environ 4-10, un ou plusieurs résidus d'acide aminé en dehors du site de liaison à la protéase, qui participent à la stabilisation de l'analogue, sont supprimés ou remplacés par un ou plusieurs autres résidus d'acide aminé, et/ou un ou plusieurs résidus d'acide aminé, qui déstabilisent l'analogue, sont ajoutés à l'extérieur du site de liaison à la protéase

**à l'exception** des analogues d'aprotinine dans lesquels l'acide aminé en position 1 ou les acides aminés en positions 1 et 2 ont été supprimés ; Ile en position 19 est remplacé par un autre résidu d'acide aminé naturel ; Lys en position 41 est remplacé par un autre résidu d'acide aminé naturel ; ou Arg en position 42 est remplacé par Lys ou Ser.

**2.** Analogue d'aprotinine selon la revendication 1, qui a la formule générale I,

$$R^1 \text{ Asp Phe Cys Leu Glu Pro Pro } R^2 \text{ Thr Gly Pro Cys Lys Ala Arg}$$
$$\text{Ile Ile } R^3 \text{ Tyr Phe Tyr } R^4 \text{ Ala } R^5 \text{ Ala Gly Leu Cys } R^6 \text{ Thr Phe Val}$$
$$\text{Tyr Gly Gly Cys Arg } R^7 \text{ } R^8 \text{ } R^9 \text{ Asn } R^{10} \text{ Phe } R^{11} \text{ Ser Ala Glu Asp Cys}$$
$$\text{Met } R^{12} \text{ Thr Cys Gly Gly Ala}$$

$$(I)$$

dans lequel

$R^1$ représente un dipeptide choisi dans l'ensemble constitué de Arg-Pro, Glu-Pro, Asp-Pro, Ala-Pro, Ile-Pro, Thr-Pro, His-Pro, Leu-Pro, Gly-Pro et Ser-Pro, Pro, ou $R^1$ représente un atome d'hydrogène,

$R^2$ représente un résidu d'acide aminé choisi dans l'ensemble constitué de Tyr, Glu, Asp, Ser, Thr, Ala et Val,

$R^3$ représente un résidu d'acide aminé choisi dans l'ensemble constitué de Arg, Glu, Asp, Leu, Ser, Ala, Gln et Thr,

$R^4$ représente un résidu d'acide aminé choisi dans l'ensemble constitué de Asn, Glu et Asp,

$R^5$ représente un résidu d'acide aminé choisi dans l'ensemble constitué de Lys, Glu, Asp, Thr, Val, Ala, Ser, Phe, Gln et Gly,

$R^6$ représente un résidu d'acide aminé choisi dans l'ensemble constitué de Gln, Glu, Asp, Val et Ala,

$R^7$ représente un résidu d'acide aminé choisi dans l'ensemble constitué de Ala, Asp, Glu et Gly,

$R^8$ représente un résidu d'acide aminé choisi dans l'ensemble constitué de Lys, Glu, Asp, Asn, Ser, Thr et Ala,

$R^9$ représente un résidu d'acide aminé choisi dans l'ensemble constitué de Arg, Glu, Asp, Ser, Asn, Leu, Gly, Gln, Met et Thr, $R^{10}$ représente un résidu d'acide aminé choisi dans l'ensemble constitué de Asn, Glu et Asp, $R^{11}$ représente un résidu d'acide aminé choisi dans l'ensemble constitué de Lys, Glu, Asp, Leu, Tyr, Ala, Val, Thr, Ser, Pro, His et Ile, et

$R^{12}$ représente un résidu d'acide aminé choisi dans l'ensemble constitué de Arg, Glu, Asp, Gln, Ala, Asn, His, Gly, Ser et Thr,

à la condition qu'au moins un des résidus d'acide aminé $R^1$-$R^{12}$ soit différent du résidu d'acide aminé correspondant d'aprotinine native et que, lorsque $R^1$ représente un atome d'hydrogène, au moins un des résidus d'acide aminé $R^2$-$R^{12}$ soit différent du résidu d'acide aminé correspondant d'aprotinine native, sauf que, lorsque $R^1$ représente un atome d'hydrogène, $R^9$ ne représente pas Ser.

3. Analogue d'aprotinine selon la revendication 1, dans lequel un peptide essentiellement constitué d'un ou plusieurs résidus d'acide aminé négativement chargés ou neutres est ajouté à l'extrémité N-terminale de la molécule d'aprotinine.

4. Analogue d'aprotinine selon la revendication 1, dans lequel un peptide essentiellement constitué d'un ou plusieurs résidus d'acide aminé négativement chargés ou neutres est ajouté à l'extrémité C-terminale de la molécule d'aprotinine.

5. Analogue d'aprotinine selon l'une quelconque des revendications précédentes, qui, en plus d'être modifié en dehors du site de liaison à la protéase, est modifié par substitution d'un ou plusieurs résidus d'acide aminé dans le site de liaison à la protéase, par un ou plusieurs autres résidus d'acide aminé, respectivement, **à l'exception** des analogues d'aprotinine dans lesquels l'acide aminé en position 1 ou les acides aminés en positions 1 et 2 ont été supprimés ; Ile en position 19 est remplacé par un autre résidu d'acide aminé naturel ; Lys en position 41 est remplacé par un autre résidu d'acide aminé naturel ; ou Arg en position 42 est remplacé par Lys ou Ser ; et dans lequel Gly en position 12 est remplacé par un autre résidu d'acide aminé naturel ; Pro en position 13 est remplacé par un autre résidu d'acide aminé naturel ; Cys en position 14 est remplacé par un autre résidu d'acide aminé naturel ; Lys en position 15 est remplacé par Arg, Val, Thr, Ile, Leu, Phe, Gly, Ser, Met, Trp, Tyr ou Ala ; Ala en position 16 est Gly ; Arg en position 17 est remplacé par un autre résidu d'acide aminé naturel ; Ile en position 18 est remplacé par Leu, Met, Val ou Phe ; ou Cys en position 38 est remplacé par un autre résidu d'acide aminé naturel.

6. Analogue d'aprotinine selon la revendication 5, qui a la formule générale II

```
R¹ Asp Phe Cys Leu Glu Pro Pro R² Thr Gly Pro Cys R¹³ R¹⁴ R¹⁵ R¹⁶
R¹⁷ R³ Tyr Phe Tyr R⁴ Ala R⁵ Ala Gly Leu Cys R⁶ Thr Phe R¹⁸ Tyr R¹⁹
Gly Cys R²⁰ R⁷ R⁸ R⁹ Asn R¹⁰ Phe R¹¹ Ser Ala Glu Asp Cys Met R¹² Thr
Cys Gly Gly Ala
```

$$(II)$$

dans laquelle $R^1$-$R^{12}$ sont tels que définis dans la revendication 2,

$R^{13}$ représente un résidu d'acide aminé choisi dans l'ensemble constitué de Lys, Arg, Glu, Leu, Met, Tyr et Phe,

$R^{14}$ représente un résidu d'acide aminé choisi dans l'ensemble constitué de Ala et Gly,

$R^{15}$ représente un résidu d'acide aminé choisi dans l'ensemble constitué de Arg, Ala, Gly, Lys, Leu, Met, Phe, Tyr, Ile et Asn,

$R^{16}$ représente un résidu d'acide aminé choisi dans l'ensemble constitué de Ile, Met, Leu, Phe, Thr et Glu,

$R^{17}$ représente un résidu d'acide aminé choisi dans l'ensemble constitué de Ile, Leu, Lys, Gln, Glu, Ser, Arg, Thr et Asn,

$R^{18}$ représente un résidu d'acide aminé choisi dans l'ensemble constitué de Val, Thr, Leu, Ser, Tyr, Gln, His, Pro, Phe, Asn, Ile et Lys,

$R^{19}$ représente un résidu d'acide aminé choisi dans l'ensemble constitué de Gly, Thr et Ser, et

$R^{20}$ représente un résidu d'acide aminé choisi dans l'ensemble constitué de Gln, Lys, Met, Asn, Leu, Gly et Glu,

à la condition qu'au moins un des résidus d'acide aminé $R^1$-$R^{12}$ et au moins un des résidus d'acide aminé $R^{13}$-$R^{20}$ soient différents des résidus d'acide aminé correspondants d'aprotinine native et que lorsque $R^1$ représente un atome d'hydrogène, $R^{15}$ ne représente pas Ala, $R^{17}$ ne représente pas Glu, et $R^9$ ne représente pas Ser, et que lorsque $R^{15}$ représente Ala, $R^9$ ne représente pas Ser

7. Analogue d'aprotinine selon la revendication 2, dans lequel $R^1$ représente Glu-Pro, $R^5$ représente Glu, $R^8$ représente Glu, $R^1$ représente Glu, et $R^2$, $R^3$, $R^4$, $R^6$, $R^7$, $R^9$, $R^{10}$ et $R^{12}$ sont tels que dans la séquence d'aprotinine native.

8. Analogue d'aprotinine selon la revendication 2, dans lequel $R^1$ représente Glu-Pro, $R^9$ représente Glu, $R^{11}$ représente Glu, et $R^2$, $R^3$, $R^4$, $R^6$, $R^7$, $R^8$, $R^{10}$ et $R^{12}$ sont tels que dans la séquence d'aprotinine native.

9. Analogue d'aprotinine selon la revendication 2, dans lequel $R^9$ représente Glu, $R^{11}$ représente Glu, et $R^1$, $R^2$, $R^3$, $R^4$, $R^5$ $R^6$, $R^7$, $R^8$, $R^{10}$ et $R^{12}$ sont tels que dans la séquence d'aprotinine native.

10. Analogue d'aprotinine selon la revendication 2, dans lequel $R^2$ représente Ser, $R^4$ représente Asp, $R^5$ représente Thr, $R^6$ représente Glu, $R^8$ représente Asn, $R^{12}$ représente Glu, et $R^1$, $R^3$, $R^7$, $R^9$, $R^{10}$ et $R^{11}$ sont tels que dans la séquence d'aprotinine native. lequel $R^2$

11. Analogue d'aprotinine selon la revendication 2, dans lequel $R^2$ représente Ser, $R^3$ représente Leu, $R^7$ représente Gly, $R^8$ représente Asn, $R^9$ représente Gly, $R^{10}$ représente Glu, $R^{11}$ représente Tyr et $R^1$, $R^4$, $R^5$, $R^6$, et $R^{12}$ sont tels que dans la séquence d'aprotinine native.

12. Analogue d'aprotinine selon la revendication 2, dans lequel $R^1$ représente un atome d'hydrogène, $R^9$ représente Ser, $R^{11}$ représente Glu, et $R^2$, $R^3$, $R^4$, $R^5$, $R^6$, $R^7$, $R^8$, $R^{10}$ et $R^{12}$ sont tels que dans la séquence d'aprotinine native.

13. Analogue d'aprotinine selon la revendication 2, dans lequel $R^1$ représente un atome d'hydrogène, $R^9$ représente Ser, $R^{11}$ représente Ala, et $R^2$, $R^3$, $R^4$, $R^5$, $R^6$, $R^7$, $R^8$, $R^{10}$ et $R^{12}$ sont tels que dans la séquence d'aprotinine native.

14. Analogue d'aprotinine selon la revendication 2, dans lequel $R^1$ représente un atome d'hydrogène, $R^2$ représente Ser, $R^4$ représente Asp, $R^5$ représente Thr, $R^6$ représente Glu, $R^8$ représente Asn, $R^{12}$ représente Glu et $R^3$, $R^7$, $R^9$, $R^{10}$ et $R^{11}$ sont tels que dans la séquence d'aprotinine native.

15. Analogue d'aprotinine selon la revendication 2, dans lequel $R^1$ représente un atome d'hydrogène, $R^4$ représente Asp, $R^5$ représente Thr, $R^6$ représente Glu, $R^{12}$ représente Glu et $R^2$, $R^3$, $R^7$, $R^8$, $R^9$, $R^{10}$ et $R^{11}$ sont tels que dans la séquence d'aprotinine native.

16. Analogue d'aprotinine selon la revendication 2, dans lequel $R^1$ représente un atome d'hydrogène, $R^2$ représente Ser, $R^7$ représente Gly, $R^8$ représente Asn, $R^9$ représente Gly, $R^{12}$ représente Glu et $R^3$, $R^4$, $R^5$, $R^6$, $R^{10}$ et $R^{11}$ sont tels que dans la séquence d'aprotinine native.

17. Analogue d'aprotinine selon la revendication 2, dans lequel $R^1$ représente un atome d'hydrogène, $R^9$ représente Ser, $R^{12}$ représente Glu, et $R^2$, $R^3$, $R^4$, $R^5$, $R^6$, $R^7$, $R^8$, $R^{10}$ et $R^{11}$ sont tels que dans la séquence d'aprotinine native.

18. Analogue d'aprotinine selon la revendication 2, dans lequel $R^1$ représente un atome d'hydrogène, $R^9$ représente Glu, R12 représente Glu, et $R^2$, $R^3$, $R^4$, $R^5$, $R^6$, $R^7$, $R^8$, $R^{10}$ et $R^{11}$ sont tels que dans la séquence d'aprotinine native.

19. Analogue d'aprotinine selon la revendication 2, dans lequel $R^1$ représente un atome d'hydrogène, $R^5$ représente Glu, $R^9$ représente Ser, $R^{12}$ représente Glu, et $R^2$, $R^3$, $R^4$, $R^6$, $R^7$, $R^8$, $R^{10}$ et $R^{11}$ sont tels que dans la séquence d'aprotinine native.

20. Analogue d'aprotinine selon la revendication 2, dans lequel $R^1$ représente un atome d'hydrogène, $R^5$ représente Glu, $R^9$ représente Glu, $R^{12}$ représente Glu, et $R^2$, $R^3$, $R^4$, $R^6$, $R^7$, $R^8$, $R^{10}$ et $R^{11}$ sont tels que dans la séquence d'aprotinine native.

21. Construction d'ADN comprenant une séquence d'ADN codant pour un analogue d'aprotinine selon l'une quelconque des revendications 1-20.

22. Vecteur d'expression recombinant comprenant une construction d'ADN selon la revendication 21.

23. Cellule contenant un vecteur d'expression selon la revendication 22.

24. Méthode de production d'un analogue d'aprotinine selon l'une quelconque des revendications 1-20, dans laquelle on cultive une cellule selon la revendication 23 dans des conditions entraînant l'expression de l'analogue d'aprotinine, et on récupère l'analogue obtenu à partir de la culture.

25. Composition pharmaceutique comprenant un analogue d'aprotinine selon l'une quelconque des revendications 1-20 avec un véhicule ou un excipient pharmaceutiquement acceptable.

26. Utilisation d'un analogue d'aprotinine selon l'une quelconque des revendications 1-20 pour la fabrication d'un médicament avec une néphrotoxicité réduite par rapport à celle de l'aprotinine native.

27. Utilisation d'un analogue d'aprotinine selon l'une quelconque des revendications 1-20 pour la fabrication d'un médicament qui, lorsqu'on l'administre, entraîne une fréquence réduite de réactions anaphylactoïdes par rapport à celle observée avec l'aprotinine native.

```
                                  1              5
MetAlaGluArgLeuGluLysArgGluProAspPheCysleuGluProPro-
NcoI
CATGGCTGAGAGATTGGAGAAGAGAGAGCCTGATTTCTGTTTGGAACCTCCA-
    CGACTCTTCAACCTCTTCTCTCTCGGACTAAAGACAAACCTTGGAGGT-


     10             15             20             25
TyrThrGlyProCysLysAlaArgIleIleArgTyrPheTyrAsnAlaGlu-
         AvaII
TACACTGGTCCATGTAAAGCTAGAATCATCAGATACTTCTACAACGCCGAA-
ATGTGACCAGGTACATTTCGATCTTAGTAGTCTATGAAGATGTTGCGGCTT-


        30             35             40
AlaGlyLeuCysGlnThrPheValTyrGlyGlyCysArgAlaGluArgAsn-

GCTGGTTTGTGTCAAACTTTCGTTTACGGTGGCTGCAGAGCTGAAAGAAAC-
CGACCAAACACAGTTTGAAAGCAAATGCCACCGACGTCTCGACTTTCTTTG-


        45             50             55       58
AsnPheGluSerAlaGluAspCysMetArgThrCysGlyGlyAlaStop
                                               XbaI
AACTTCGAATCTGCTGAAGACTGCATGAGAACTTGTGGTGGTGCCTAAT
TTGAAGCTTAGACGACTTCTGACGTACTCTTGAACACCACCACGGATTAGATC
```

# Fig. 1

Fig. 2

Fig. 3

Fig. 4

Fig. 5

Fig. 6